# EUROPEAN PATENT SPECIFICATION

(11) **EP 0 315 153 B1**
(45) Date of publication and mention of the grant of the patent: **11.05.1994**
(21) Application number: 88118263.8
(22) Date of filing: 02.11.1988
(51) Int. Cl.: A61K 39/39

(54) **Vaccine adjuvant**
Impfstoff-Adjuvans
Adjuvant pour vaccin

(30) Priority: 03.11.1987 US 116425
(43) Date of publication of application: 10.05.1989
(60) Divisional application: 92113037.3
(73) Proprietor: SYNTEX (U.S.A.) INC., Palo Alto California 94303 (US)
(72) Inventor: Allison, Anthony Clifford, Belmont, CA 94002 (US); Byars, Noelene Elva, Sunnyvale, CA 94087 (US); Fu, Cherng-Chyi, Saratoga, CA 95070 (US); Lidgate, Deborah Marilyn, Los Altos, CA 94022 (US); Felgner, Philip Lewis, Rancho Santa Fe, CA 92067 (US); Foster, Linda Cheryl, Sunnyvale, CA 94086 (US); Lee, William Alfred, Los Altos, CA 94022 (US)
(74) Representative: Barz, Peter, Dr.

(56) References cited:
- EP-A- 0 135 376
- WO-A-88/01873
- THE JOURNAL OF IMMUNOLOGY, vol. 127, no. 3 September 1981, The American Association of Immunologists; R. HUNTER et al., pp. 1244-1250#
- VACCINE, vol. 5, no. 3, September 1987, Butterworth & Co. Ltd., London (GB); N.E. BYARS et al., pp. 223-228#

## Description

This invention relates to improved vaccine adjuvant formulations, improved processes for preparing said adjuvant formulations, and methods of using the improved formulations.

Adjuvants are useful for improving the immune response obtained with any particular antigen in a vaccine. Although some antigens are administered in vaccines without an adjuvant, there are many antigens that lack sufficient immunogenicity to stimulate a useful immune response in the absence of an effective adjuvant. Adjuvants also improve the immune response obtained from "self-sufficient" antigens, in that the immune response obtained may be increased or the amount of antigen administered may be reduced.

The standard adjuvant for use in laboratory animals is Freund's adjuvant. Freund's complete adjuvant (FCA) is an emulsion containing mineral oil and killed mycobacteria in saline. Freund's incomplete adjuvant (FIA) omits the mycobacteria. Both FIA and FCA induce exceptional humoral (antibody) immunity, and FCA additionally induces high levels of cell-mediated immunity. However, neither FIA nor FCA are acceptable for use outside the laboratory due to the adjuvants' side effects. Mineral oil is known to cause abscesses and granulomas, while Mycobacterium tuberculosis is the agent responsible for tuberculosis.

A number of naturally occurring compounds such as the lipid-A portion of gram negative bacteria endotoxin and trehalose dimycolate of mycobacteria have been tried as substitutes for FCA and FIA. Also, the phospholipid lysolecithin has been shown to have adjuvant activity (B. Arnold et al., Eur. J. Immunol., 9:363-366 (1979)). In addition, several synthetic surfactants, for example, dimethyldioctadecyl ammonium bromide (DDA) and certain linear polyoxypropylene-polyoxyethylene (POP-POE) block polymers (available commercially under the trademark Pluronic®) have been reported as having adjuvant activity (H. Snippe et al, Int. Archs. Allergy Appl. Immun., 65, 390-398 (1981)). R. Hunter et al. have reported in J. Immunol., 127, 1244-1250 (1981) that POP-POE block polymers increase antibody formation to bovine serum albumin (BSA) in mice when used as the surfactant component of an mineral oil/water emulsion adjuvant formulation. While these natural and synthetic surfactants demonstrate some degree of adjuvanticity, they for the most part fail to achieve the degree of immunopotentiation obtained using FCA or FIA.

Taking another approach, it has been determined that the adjuvant effect from mycobacteria is due to a muramyl-peptide in the cell wall. The smallest fragment of this molecule that retains adjuvant activity is N-acetylmuramyl-L-alanyl-D-isoglutamine, commonly known as muramyldipeptide or "MDP" (Ellouz et al, Biochem. & Biophys. Res. Comm., Vol 59, 4, 1317 (1974)). Numerous analogs of MDP have been prepared, and are also referred to as "MDPs." See for example Audibert et al., US-A-4,158,052; Audibert et al., US-A-4,220,637; Audibert et al., US-A-4,323,559; Baschang et al., US-A-4,323,560; Baschang et al., US-A-4,409,209; Baschang et al., US-A-4,423,038; Derrien et al., US-A-4,185,089; Hartmann et al., US-A-4,406,889; Jones et al., US-A-4,082,735; Jones et al., US-A-4,082,736; Le Francier et al., US-A-4,427,659; Le Francier et al., US-A-4,461,761; Yamamura et al., US-A-4,314,998; Yamamura et al., US-A-4,101,536; and Yamamura et al., US-A-4,369,178. While these compounds are weakly effective at stimulating the immune system when administered in aqueous solution, the results generally fall short of the specific immune response obtained with FIA or FCA.

A particularly effective adjuvant formulation comprising a glycopeptide, a non-toxic POP-POE block polymer, a glycol ether-based surfactant, a metabolizable oil, and buffered saline was recently described by Allison et al., US-A-4,606,918. This formulation is capable of inducing strong humoral and cell-mediated immune responses, equivalent or superior to the results achieved in laboratory animals using FCA. However, the formulation is prone to instability and separation (e.g., creaming) upon standing. We have discovered that upon refrigeration it loses its ability to potentiate the primary response to antigens. Also, it has been found difficult to prepare a stable, homogenous emulsion with retention of full adjuvant activity on a commercial scale.

We have now discovered that an immunopotentiating glycopeptide can be formulated with a non-toxic N,N,N',N'-tetra(polyoxypropylene-polyoxyethylene)-1,2-diaminoethane block polymer ("tetra-polyol"), resulting in an adjuvant emulsion that overcomes certain problems of the prior art, for example, toxicity and failure to stimulate cell-mediated immunity. This new adjuvant has activity equal or greater than the activity of FCA and Allison's composition. The formulation of the present invention is easily manufactured with full retention of activity, and displays greater stability than Allison's composition. Because the tetra-polyol is non-toxic, this adjuvant formulation may be safely used as a vehicle for enhancing the immunogenicity of antigens administered to birds and mammals.

We have also invented a particularly advantageous method for preparing adjuvant emulsions where substantially all the oily particles have a diameter less than about 800 nm, using either POP-POE block polymers or tetra-polyols, which maintains the formulations' efficacy, enhances its physical stability, and reduces its sensitivity to refrigeration. Remarkably, such adjuvant emulsions may even be frozen and still retain efficacy.

One aspect of the invention is an adjuvant in the form of an emulsion having oily particles dispersed in a continuous aqueous phase, for potentiating the immunogenicity of an antigen, which adjuvant comprises:
an emulsion-forming amount of a non-toxic tetra-polyol or of a POP-POE block polymer; and
an immunopotentiating amount of a glycopeptide;
wherein substantially all of said oily particles have a diameter less than about 800 nm.

Another aspect of the invention is an adjuvant in the form of an emulsion having oily particles dispersed in a continuous aqueous phase, for potentiating the immunogenicity of an antigen, which adjuvant comprises an emulsion-forming amount of a non-toxic tetra-polyol; optionally, an emulsion-forming amount of a non-toxic metabolizable oil; optionally, an emulsion-stabilizing amount of a glycol ether-based surfactant; water or aqueous solution, and an immunopotentiating amount of a muramyldipeptide, preferably a derivative of formula I 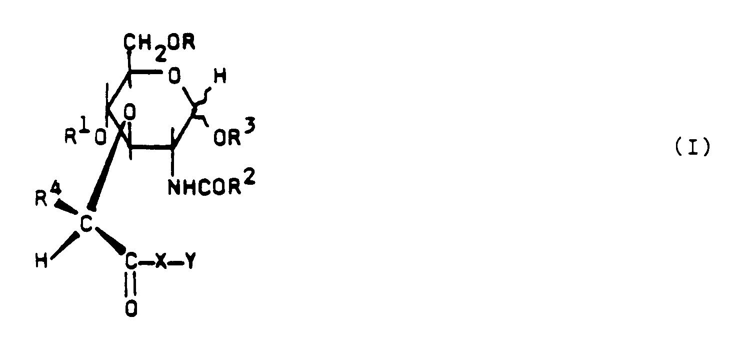
and the pharmaceutically acceptable salts thereof, wherein R and R₁ are each independently H or acyl of 1 to 22 carbon atoms, R₂ is alkyl or aryl, optionally substituted with halo, nitro, or lower alkyl, R₃ is H, alkyl, or aryl, R₄ is H or lower alkyl, X is L-alanyl, L-α-aminobutyryl, L-arginyl, L-asparginyl, L-aspartyl, L-cysteinyl, L-glutaminyl, L-glutamyl, glycyl, L-histidyl, L-hydroxyprolyl, L-isoleucyl, L-leucyl, L-lysyl, L-methionyl, L-ornithinyl, L-phenylalanyl, L-prolyl, L-seryl, L-threonyl, L-tyrosyl, L-tryptophanyl, or L-valyl, and Y is D-glutamine, D-isoglutamine or D-isoasparagine.

Another aspect of the invention is an adjuvant of the type mentioned above, where an emulsion-forming amount of a non-toxic POP-POE block polymer may be substituted for the tetra-polyol, and where substantially all of the oily particles have a diameter less than about 800 nm, preferably less than 300 nm.

Another aspect of the invention is a vaccine, comprising an adjuvant of the invention in combination with an immunogenic amount of an antigen.

Another aspect of the invention is a process for preparing an adjuvant of the invention, which process comprises preparing a first mixture comprising the polymer, oil, surfactant, and water or aqueous solution; emulsifying the mixture to produce an oil-in-water type emulsion having oily particles dispersed in a continuous aqueous phase, wherein substantially all of said oily particles have a diameter less than about 800 nm, preferably less than about 300 nm; and combining the emulsion with a muramyldipeptide derivative of formula I.

Another aspect of the invention is a kit for extemporaneous preparation of an adjuvant of the invention, which kit comprises: a first container containing an emulsion as described above, and a second container containing the muramyl dipeptide, preferably N-acetylmuramyl-L-threonyl-D-isoglutamine optionally in an aqueous solution or suspension, where the concentrations of the components in each container are selected such that combination of the contents of both containers produces an adjuvant of the invention.

Another aspect of the invention is a kit for the preparation of a vaccine of the invention, which differs from the adjuvant kit described above in that an immunogenic amount of an antigen is added to the second container, or present in a third container.

One aspect of the invention is an adjuvant in the form of an emulsion having oily particles dispersed in a continuous aqueous phase, for potentiating the immunogenicity of an antigen, which adjuvant comprises:
an emulsion-forming amount of a non-toxic tetra-polyol or of a POP-POE block,polymer; and
an immunopotentiating amount of a glycopeptide;
wherein substantially all of said oily particles have a diameter less than about 800 nm.

Another aspect of the invention is an adjuvant in the form of an emulsion having oily particles dispersed in a continuous aqueous phase, for potentiating the immunogenicity of an antigen, which adjuvant comprises an emulsion-forming amount of a non-toxic tetra-polyol; optionally, an emulsion-forming amount of a non-toxic metabolizable oil; optionally an emulsion-stabilizing amount of a glycol ether-based surfactant; water or aqueous solution, and an immunopotentiating amount of a muramyldipeptide, preferably a derivative of formula I 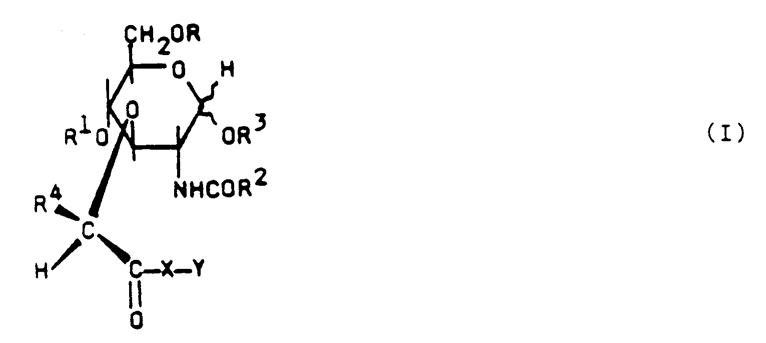
and the pharmaceutically acceptable salts thereof, wherein R and R₁ are each independently H or acyl of 1 to 22 carbon atoms, R₂ is alkyl or aryl, optionally substituted with halo, nitro, or lower alkyl, R₃ is H, alkyl, or aryl, R₄ is H or lower alkyl, X is L-alanyl, L-α-aminobutyryl, L-arginyl, L-asparginyl, L-aspartyl, L-cysteinyl, L-glutaminyl, L-glutamyl, glycyl, L-histidyl, L-hydroxyprolyl, L-isoleucyl, L-leucyl, L-lysyl, L-methionyl, L-ornithinyl, L-phenylalanyl, L-prolyl, L-seryl, L-threonyl, L-tyrosyl, L-tryptophanyl, or L-valyl, and Y is D-glutamine, D-isoglutamine or D-isoasparagine. A preferred subgenus is the adjuvant wherein said tetra-polyol is Tetronic® 1501, particularly where said muramyldipeptide derivative of formula I is N-acetylmuramyl-L-threonyl-D-isoglutamine. A preferred class of the invention is the adjuvant which includes a non-toxic metabolizable oil, especially where said oil is squalene or squalane. A preferred subclass is the adjuvant wherein said glycol ether-based surfactant is polysorbate 80 (Tween® 80), particularly where said water or aqueous solution comprises isotonic buffered saline, and especially where substantially all of the oily particles have a diameter less than about 800 nm, preferably less than about 300 nm. Another preferred subgenus is the adjuvant wherein said tetra-polyol is Tetronic® 1501 and said muramyldipeptide derivative of formula I is murabutide.

Another aspect of the invention is an adjuvant in the form of an oil-in-water type emulsion, having oily particles dispersed in a continuous aqueous phase, for potentiating the immunogenicity of an antigen, which adjuvant comprises a non-toxic tetra-polyol in an amount of 0.2 to 49%; a non-toxic metabolizable oil in an amount of 0-15%; a glycol ether-based surfactant in an amount of 0.05-5%; water or aqueous solution; and 0.0001-10% of a muramyldipeptide derivative of formula I (% are vol./vol., except for the muramyldipeptide which is wt./vol.). A preferred subgenus is the adjuvant wherein said tetra-polyol is Tetronic® 1501, particularly where said muramyldipeptide derivative of formula I is N-acetyl-muramyl-L-threonyl-D-isoglutamine. A preferred class is the adjuvant which includes a non-toxic metabolizable oil, wherein said oil is squalene or squalane. A presently preferred embodiment of the invention is the adjuvant in the form of an oil-in-water type emulsion, having oily particles dispersed in a continuous aqueous phase, for potentiating the immunogenicity of an antigen, which adjuvant comprises Tetronic® 1501 in an amount of 1-10%; squalane or squalene in an amount of 1-10%; Tween® 80 in an amount of about 0.2%; isotonic buffered saline (using phosphate buffers, acetate buffers, or combinations thereof); and 0.0001-10% N-acetylmuramyl-L-threonyl-D-isoglutamine, particularly where substantially all of said oily particles have a diameter less than about 800 nm, preferably less than about 300 nm.

Another aspect of the invention is an adjuvant in the form of an oil-in-water type emulsion, having oily particles dispersed in a continuous aqueous phase, for potentiating the immunogenicity of an antigen, which adjuvant comprises an emulsion-forming amount of a non-toxic POP-POE block polymer; optionally, an emulsion-forming amount of a non-toxic metabolizable oil; an emulsion-stabilizing amount of a glycol ether-based surfactant; water or aqueous solution; and an immunopotentiating amount of a muramyldipeptide derivative of formula I, wherein substantially all of said oily particles have a diameter less than about 800 nm, preferably less than about 300 nm. A preferred subgenus is the adjuvant wherein said non-toxic POP-POE block polymer is Pluronic® L121, particularly where said muramyldipeptide derivative of formula I is N-acetylmuramyl-L-threonyl-D-isoglutamine. A preferred class is the adjuvant which includes a non-toxic metabolizable oil, wherein said oil is squalene or squalane. A preferred subclass is the adjuvant wherein said glycol ether-based surfactant is Tween® 80, particularly where said water or aqueous solution comprises isotonic buffered saline.

Another aspect of the invention is an adjuvant in the form of an oil-in-water type emulsion, having oily particles dispersed in a continuous aqueous phase, for potentiating the immunogenicity of an antigen, which adjuvant comprises a non-toxic POP-POE block polymer in an amount of 0.2 to 49%; a non-toxic metabolizable oil in an amount of 0-15%; a glycol ether-based surfactant in an amount of 0.05-5%; water or aqueous solution; and 0.0001-10% of a muramyldipeptide derivative of formula I, where substantially all of said oily particles have a diameter less than about 800 nm, preferably less than about 300 nm (% are vol./vol., except for the muramyldipeptide which is wt./vol.). A preferred subgenus is the adjuvant wherein said POP-POE block polymer is Pluronic® L121, particularly where said muramyldipeptide derivative of formula I is N-acetylmuramyl-L-threonyl-D-isoglutamine. A preferred class is the adjuvant which includes a non-toxic metabolizable oil, wherein said oil is squalene or squalane. A preferred subclass is the adjuvant wherein said glycol ether-based surfactant is Tween® 80. A presently preferred embodiment is the adjuvant in the form of an oil-in-water type emulsion, having oily particles dispersed in a continuous aqueous phase, for potentiating the immunogenicity of an antigen, which adjuvant comprises Pluronic® L121 in an amount of 1-10%; squalane or squalene in an amount of 1-10%; Tween® 80 in an amount of about 0.2%; isotonic buffered saline; and 0.0001-10% N-acetylmuramyl-L-threonyl-D-isoglutamine, wherein substantially all of said oily particles have a diameter less than about 800 nm, preferably less than about 300 nm.

Another aspect of the invention is a vaccine comprising an adjuvant of the invention in combination with an immunogenic amount of an antigen. Suitably this is a vaccine in the form of an oil-in-water type emulsion, having oily particles dispersed in a continuous aqueous phase, for immunizing an animal, which vaccine comprises an immunogenic amount of an antigen; an emulsion-forming amount of a non-toxic tetra-polyol or a non-toxic POP-POE block polymer; optionally, an emulsion-forming amount of a non-toxic metabolizable oil; optionally an emulsion-stabilizing amount of a glycol ether-based surfactant; water or aqueous solution; and an immunopotentiating amount of a muramyldipeptide, preferably a derivative of formula I. A preferred subgenus is the vaccine which includes a tetra-polyol, especially where said tetra-polyol is Tetronic® 1501. A preferred class is the vaccine wherein substantially all of said oily particles have a diameter less than about 800 nm, preferably less than about 300 nm. A preferred subclass is the vaccine wherein said muramyldipeptide derivative of formula I is N-acetylmuramyl-L-threonyl-D-isoglutamine. Another preferred subclass is the vaccine wherein said muramyldipeptide derivative of formula I is murabutide. A presently preferred embodiment is the vaccine which comprises: Tetronic® 1501 in an amount of 1-10%; squalane or squalene in an amount of 1-10%; Tween® 80 in an amount of about 0.2%; isotonic buffered saline; and 0.0001-10% N-acetylmuramyl-L-threonyl-D-isoglutamine, especially where substantially all of said oily particles have a diameter less than about 800 nm, preferably less than about 300 nm. Another preferred subgenus is the vaccine which includes a POP-POE block polymer, wherein said block polymer is Pluronic® L121, wherein substantially all of said oily particles have a diameter less than about 800 nm, preferably less than about 300 nm. A preferred class is the vaccine wherein said muramyldipeptide derivative of formula I is N-acetylmuramyl-L-threonyl-D-isoglutamine. Another preferred class is the vaccine wherein said muramyldipeptide derivative of formula I is murabutide. A presently preferred embodiment is the vaccine which comprises: Pluronic® L121 in an amount of 1-10%; squalane or squalene in an amount of 1-10%; Tween® 80 in an amount of about 0.2%; isotonic buffered saline; and 0.0001-10% N-acetylmuramyl-L-threonyl-D-isoglutamine.

Another aspect of the invention is a process for preparing the adjuvant or vaccine of the invention, which process comprises mixing together the aqueous phase and the emulsion-forming amount of the non-toxic tetra-polyol or of the POP-POE block polymer so as to form an emulsion.

Another aspect of the invention is a process for preparing an adjuvant of the invention, which process comprises: preparing a first mixture comprising a non-toxic tetra-polyol or a non-toxic POP-POE block polymer, optionally a non-toxic metabolizable oil, optionally a glycol ether- based surfactant, and water or aqueous solution; emulsifying said first mixture to produce an oil-in-water type emulsion, having oily particles dispersed in a continuous aqueous phase, wherein substantially all of said oily particles have a diameter less than about 800 nm, preferably less than about 300 nm; and combining said emulsion with a muramyldipeptide derivative of formula I. A preferred class is the process wherein said first mixture is emulsified using a Microfluidizer® (or other suitable emulsifying technique) to obtain an emulsion wherein substantially all of said oily particles have a diameter less than about 800 nm, preferably less than about 300 nm. A preferred subclass is the process wherein said muramyldipeptide derivative of formula I is combined with said emulsion in the form of an aqueous solution or suspension.

Another aspect of the invention is a kit for extemporaneous preparation of an adjuvant of the invention, which kit comprises:
a first container containing the emulsion of the tetra-polyol or POP-POE polymer in the aqueous phase where substantially all the oily particles have a diameter less than about 800 nm; and
a second container containing the glycopeptide.

Another aspect of the invention is a kit for extemporaneous preparation of a vaccine of the invention, which kit comprises:
a first container containing the emulsion of the tetra-polyol or POP-POE block polymer in the aqueous phase; where substantially all the oily particles have a diameter less than 800 nm; and
a second container containing the antigen;
wherein the glycopeptide may be present in a third container, or in the first or second containers.

Another aspect of the invention is a kit for extemporaneous preparation of an adjuvant of the invention, which kit comprises: a first container containing an oil-in-water type emulsion, having oily particles dispersed in a continuous aqueous phase, where said emulsion comprises Tetronic® 1501 or Pluronic® L121, squalane or squalene, optionally Tween® 80, and isotonic buffered saline; and a second container containing N-acetylmuramyl-L- threonyl-D-isoglutamine in powder form (preferably lyophilized) or in aqueous solution or suspension, where the concentrations of the components in each container are selected such that combination of the contents of both containers produces a formulation comprising Tetronic® 1501 or Pluronic® L121 in an amount of 1-30%, squalane or squalene in an amount of 1-30%, optionally Tween® 80 in an amount of about 0.2-5%, 0.0001-30% N-acetylmuramyl-L-threonyl-D-isoglutamine, and isotonic buffered saline. A preferred subgenus is the kit wherein Tetronic® 1501 is included. A preferred class is the kit wherein substantially all of said oily particles have a diameter less than about 800 nm, preferably less than about 300 nm. Another preferred subgenus is the kit wherein Pluronic® L121 is included.

Another aspect of the invention is a kit for extemporaneous preparation of a vaccine of the invention, which kit comprises: a first container containing an oil-in-water type emulsion, having oily particles dispersed in a continuous aqueous phase, where said emulsion comprises Tetronic® 1501 or Pluronic® L121, squalane or squalene, optionally Tween® 80, and isotonic buffered saline; and a second container containing N-acetylmuramyl-L-threonyl-D- isoglutamine in powder form (preferably lyophilized) or in aqueous solution or suspension and an immunogenic amount of an antigen; where the concentrations of the components in each container are selected such that combination of the contents of both containers produces a formulation comprising Tetronic® 1501 or Pluronic® L121 in an amount of 1-10%, squalane or squalene in an amount of 1-10%, optionally Tween® 80 in an amount of about 0.2%, 0.0001-10% N-acetylmuramyl-L-threonyl-D-isoglutamine, an immunogenic amount of an antigen, and isotonic buffered saline. Optionally, the antigen can be in a separate third container. A preferred subgenus is the kit wherein Tetronic® 1501 is included. A preferred class is the kit wherein substantially all of said oily particles have a diameter less than about 800 nm, preferably less than about 300 nm. Another preferred subgenus is the kit wherein Pluronic® L121 is included.

As noted, suitably in the kits of the invention the glycopeptide is present as a powder, preferably a lyophilized powder.

### DEFINITIONS

The term "alkyl" refers to a straight or branched radical comprised of 1 to 22 carbon atoms containing no unsaturation. Examples of alkyl are methyl, ethyl, propyl, butyl, tert-butyl, hexyl, octyl, decyl, dodecyl, eicosanyl, and the like. "Lower alkyl" refers to an alkyl radical of 1 to 7 carbon atoms, for example, methyl, ethyl, propyl, butyl, tert-butyl, hexyl, 3-methylhexyl, heptyl, and the like. "Cycloalkyl" refers to cyclopropyl, cyclobutyl, cyclopentyl, cyclohexyl, cycloheptyl, and the like.

The term "acyl" refers to radicals of the formula RCO-, where R is H or alkyl as defined above. "Lower acyl" refers to such radicals where R is H or lower alkyl. Examples of acyl include formyl, acetyl, propionyl, butyryl, pentanoyl, hexanoyl, eicosanoyl, and the like. Examples of lower acyl include formyl, acetyl, propionyl, butyryl, pentanoyl, hexanoyl, and the like.

The term "halo" as used herein refers to fluoro, chloro, bromo and iodo.

The term "alkoxy" refers to a radical of the form RO-, where R is lower alkyl or cycloalkyl as defined above.

The term "aryl" refers to aromatic radicals consisting entirely of carbon and hydrogen, containing from 6 to 12 carbon atoms. Examples of aryl groups are phenyl, naphthyl, and the like.

The term "pharmaceutically acceptable salts" refers to acid addition salts of the subject compounds which possess the desired pharmacological activity and which are neither biologically nor otherwise undesirable. These salts are formed with inorganic acids such as hydrochloric acid, hydrobromic acid, sulfuric acid, nitric acid or phosphoric acid; or organic acids such as acetic acid, propionic acid, glycolic acid, pyruvic acid, malonic acid, succinic acid, malic acid, maleic acid, fumaric acid, tartaric acid, citric acid, benzoic acid, cinnamic acid, mandelic acid, methanesulfonic acid, ethanesulfonic acid, p-toluenesulfonic acid and the like.

The term "treatment" as used herein covers any treatment of a disease in a bird or mammal, particularly a human, and includes:
(i) preventing the disease from occurring in a subject which may be predisposed to the disease but has not yet been diagnosed as having it;
(ii) inhibiting the disease, i.e., arresting its development; or
(iii) relieving the disease, i.e., causing regression of the disease. (It should be noted that vaccination may effect regression of a disease where the disease persists due to ineffective antigen recognition by the subject's immune system, where the vaccine effectively presents antigen.)

The term "optionally substituted" as applied to aryl radicals in the invention means that the radical may be unsubstituted or substituted with one to three halo, nitro, lower alkyl, or lower alkoxy groups. The optional substituents may be the same or different.

The term "muramyl dipeptide derivative" includes compounds of formula I: 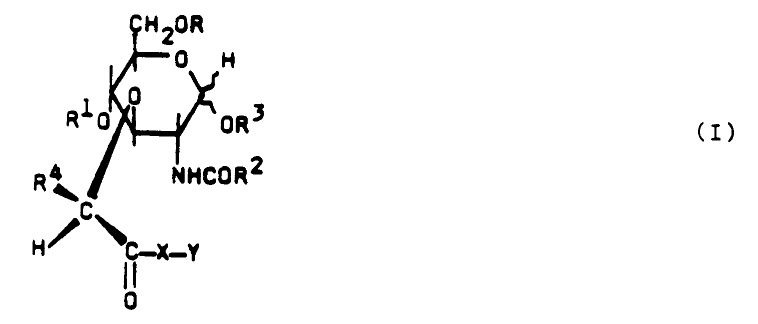
where R, R₁, R₂, R₃, and R₄ are each independently H, alkyl, acyl, or aryl optionally substituted with halo, nitro, or lower alkyl; X is one or several amino acids, and Y is D-glutamine, D-isoglutamine or D-isoasparagine, which may optionally be esterified or amidated. Preferred compounds are those of Formula 1 wherein R and R₁ are H or acyl of 1 to 22 carbon atoms; R₂ is methyl; R₃ is hydrogen; X is L-alanyl, L-α-aminobutyryl, L-arginyl, L-asparginyl, L-aspartyl, L-cysteinyl, L-glutaminyl, L-glutamyl, glycyl, L-histidyl, L-hydroxyprolyl, L-isoleucyl, L-leucyl, L-lysyl, L-methionyl, L-ornithinyl, L-phenylalanyl, L-prolyl, L-seryl, L-threonyl, L-tyrosyl, L-tryptophanyl, or L-valyl, and Y is D-glutamine or D-isoglutamine. The most preferred MDPs are: N-acetylmuramyl-L-α-aminobutyryl-D-isoglutamine; 6-0-stearoyl-N-acetylmuramyl-L-α-aminobutyryl-D-isoglutamine; N-acetylmuramyl-L-threonyl-D-isoglutamine; N-acetylmuramyl-L-valyl-D-isoglutamine; N-acetylmuramyl-L-alanyl-D-isoglutamine; N-acetyl-desmethylmuramyl-L-alanyl-D-isoglutamine; N-acetylmuramyl-L-alanyl-D-glutamine butyl ester (murabutide); N-acetylmuramyl-L-seryl-D-isoglutamine; and N-butyrylmuramyl-L-(α-aminobutyryl)-D-isoglutamine. Another useful MDP is N-acetyl-(n-butylmuramyl)-L-α-aminobutyryl-D-isoglutamine.

The term "immunopotentiating amount" refers to the amount of MDP derivative needed to effect an increase in antibody titer and/or cell mediated immunity when administered with an antigen in the formulation of the invention, as compared with the titer level observed in the absence of the MDP. As can be appreciated, each MDP may have an effective dose range that may differ from other MDPs. Thus, a single dose range cannot be prescribed which will have a precise fit for each possible glycopeptide within the scope of this invention. However, the immunopotentiating amount may easily be determined by one of ordinary skill in the art. As a general rule, the glycopeptide will preferably be present in an amount of between 0.001 and 2%. A more preferred amount is 0.005 to 1%.

The term "non-toxic metabolizable oil" refers to an oil of 6 to 30 carbon atoms including, but not limited to, alkanes, alkenes, alkynes, and their corresponding acids and alcohols, the ethers and esters thereof, and mixtures thereof. The oil may be any vegetable oil, fish oil, animal oil or synthetically prepared oil which can be metabolized in the body of the subject to which the adjuvant is administered, and which is not toxic to the organism. It is essential that the oil be metabolized by the animal or bird to which it is administered to avoid causing abscesses, granulomas or carcinomas. Nuts, seeds and grains are common sources of vegetable oils. Synthetic oils within the scope of this invention include "Neobee®" (available from PVO International, Inc., Chemical Specialities Division, 416 Division Street, Boongon, New Jersey) and others. Shark liver oil contains a branched, unsaturated oil known as squalene, 2,6,10,15,19,23-hexamethyl-2,6,10,14,18,22-tetracosahexene which is particularly preferred herein. Squalane, the saturated analog of squalene is also a particularly preferred oil. Fish oils, including squalene and squalane, are readily available from commercial sources or may be obtained by methods known in the art.

An "emulsion-forming amount" of a non-toxic metabolizable oil is that amount which will form an emulsion in the presence of the tetra-polyol or POP-POE block polymer. The oil component of these adjuvants and vaccine formulations will usually be present in an amount between 1 to 30%, but preferably in an amount of 1 to 10%. It is most preferred to use about a 5% concentration of oil.

The term "substantially all" as applied to the size of the oily emulsion particles means that greater than 70% of the particles are below the size stated (e.g., 800 nm or 300 nm), preferably 80% of the particles, and most preferably 95% or more.

The aqueous portion of these adjuvant compositions is preferably buffered isoosmotic saline. Because these compositions are intended for parenteral administration, it is preferred to formulate these solutions so that the tonicity is essentially the same as normal physiological fluids in order to prevent post-administration swelling or rapid absorption of the composition due to differential ion concentrations between the composition and physiological fluids. It is also preferred to buffer the saline in order to maintain a pH compatible with normal physiological conditions. Also, in certain instances, it may be necessary to maintain the pH at a particular level in order to insure the stability of certain composition components, such as the glycopeptides. Any physiologically acceptable buffer may be used herein, but it has been found that it is most convenient to use a phosphate buffer. Any other acceptable buffer such as acetate, Tris, bicarbonate, carbonate, and the like can be used as a substitute for a phosphate buffer. It is preferred to use phosphate buffered saline, or saline buffered with a mixture of phosphate and acetate.

The term "antigen" refers to any substance, usually a protein or glycoprotein, lipoprotein, saccharide, polysaccharide or lipopolysaccharide, which upon administration stimulates the formation of specific antibodies and reacts specifically in vivo or in vitro with a homologous antibody. Moreover, it stimulates the proliferation of T-lymphocytes with receptors for the antigen, and can react with the lymphocytes to initiate the series of responses designated cell-mediated immunity.

The term "antigen" as used herein also includes combinations of haptens with a carrier. A hapten is a portion of an antigenic molecule or antigenic complex that determines its immunological specificity, but is not sufficient to stimulate an immune response in the absence of a carrier. Commonly, a hapten is a relatively small peptide or polysaccharide and may be a fragment of a naturally occurring antigen. In artificial antigens, it may be a low molecular weight substance such as, for example, an arsanilic acid derivative. A hapten will react specifically in vivo and in vitro with homologous antibodies or T-lymphocytes. Haptens are typically attached to a large carrier molecule such as bovine serum albumin (BSA) or keyhole limpet hemocyanin (KLH) by either covalent or non-covalent binding before formulation as a vaccine. For example, a common artificial antigen used to test vaccines and adjuvants consists of 2,4-dinitrophenol (DNP) covalently bound to BSA. Suitable antigens for use in this invention include hepatitis B (surface) antigen, and influenza (for example, A or B) antigen. Also, antigens for AIDS and herpes.

The term "immunogenic amount" of an antigen refers to an amount of antigen sufficient to stimulate a useful immune response, when administered with an adjuvant of the invention. The amount of antigen necessary to provide an immunogenic amount is readily determined by one of ordinary skill in the art, e.g., by preparing a series of vaccines of the invention with varying concentrations of antigen, administering the vaccines to suitable laboratory animals (e.g., Guinea pigs), and assaying the resulting immuno response by measuring serum antibody titer, antigen-induced swelling in the skin, and the like.

The term "tetra-polyol" as used herein refers to N,N,N′,N′-tetra(polyoxypropylene-polyoxyethylene)-1,2-diaminoethane block polymers. These compounds may be prepared by the process disclosed in US-A-2,979,528, or may be obtained commercially from BASF-Wyandotte under the trademark Tetronic®.

Tetronic® polyols are designated with a three or four digit number which indicates the average molecular weight of the polyoxypropylene (POP) portion and the percentage of the total molecular weight contributed by the polyoxyethylene (POE) portion of the molecule. The first one or two non-zero digits indicate the average molecular weight of the POP section, ranging from 501-1000 for Tetronic® 304 to 6500-7000 for Tetronic® 1501. The last digit indicates the percentage of POE in 10% increments, ranging from 10% for Tetronic® 1501 to 80% for Tetronic® 1508. The characteristics of these compounds are determined by the molecular weight of the POP portion and the amount of POE in the product. Preferred tetra-polyols in the practice of the invention are relatively insoluble in water at 25°C and have low HLB values. The HLB value should preferably be lower than about 5.0. Presently preferred tetra-polyols are Tetronic® 1501, Tetronic® 1301, Tetronic® 1101, and Tetronic® 1502, particularly Tetronic® 1501 and Tetronic® 1301, especially Tetronic® 1501. Other appropriate tetra-polyols with the necessary properties may be prepared using the methods disclosed in US-A-2,979,528, and are to be considered equivalents within the scope of this invention. For example, one could prepare a tetra-polyol with a POP molecular weight of 8,000 and a POE content of 8%. The properties necessary are (i) low HLB value ( ≦ 5.0, preferably ≦ 2.0); (ii) little or no aqueous solubility; (iii) forms stable emulsions with the addition of a glycol ether-based surfactant; and (iv) lack of toxicity.

The term "POP-POE block polymer" refers to a polymer made by the sequential addition of propylene oxide and then ethylene oxide to a low molecular weight, reactive compound, usually propylene glycol. These block polymers can be prepared by the methods set out in US-A-2,674,619 issued to Lunsted, and are commercially available from BASF-Wyandotte under the trademark Pluronic®. The characteristics of these polyols are determined by the molecular weight of the POP nucleus and of the percentage POE in the product. The POP section imparts hydrophobic characteristics to the block polymer, while the POE section imparts hydrophilic characteristics. Preferred block polymers are determined by the same criteria used to select appropriate tetra-polyols. Preferred block polymers for the practice of the invention are Pluronic® L121 and L101.

Pluronic® polyols are designated by a letter prefix followed by a two or a three digit number. The letter prefixes (L, P, or F) refer to the physical form of each polymer, (liquid, paste, or flakeable solid). The first one or two digits is a code for the average molecular weight of the POP base, while the last digit indicates the amount of POE. For example, Pluronic® L101 is a liquid having a polyoxypropylene base of average molecular weight 3,250, with 10% polyoxyethylene present at the ends of the molecule. The preferred block polymers are those which are liquid over a temperature range between about 15°-40°C. In addition, polymer mixtures of liquid and paste, liquid, paste and flakeable solid or liquid and flakeable solid mixtures which are liquid within the specified temperature range may have utility in this invention.

Preferred block polymers are those having a POP base ranging in molecular weight between about 2250 and 4300 and POE in an amount between about 1 and 30%. More preferred are those polymers wherein POP has a molecular weight falling between 3250 and 4000 and the POE component comprises 10-20%. The Pluronic® polyols L101, L121 and L122 fall within this definition. Most preferred are the polymers wherein POP has a molecular weight of 4000 and POE in an amount of 10% or POP has a molecular weight of 3250 and POE in an amount of 10% eg. Pluronic® polyols L121 and L101 respectively.

An "emulsion-forming amount" of tetra-polyol or POP-POE block polymer is that quantity which will form micelles or an emulsion. For the purposes of the invention this is an amount between 0.2% and 49% by volume. A more preferred amount is from 0.2% to 20%, and about 1-5% is even more preferred. A concentration of 1-2.5% is presently most preferred.

The term "surfactant" refers to non-toxic surface active agents capable of stabilizing the emulsion. There are a substantial number of emulsifying and suspending agents generally used in the pharmaceutical sciences. These include naturally derived materials such as gums, vegetable protein, alginates, cellulose derivates, phospholipids (whether natural or synthetic), and the like. Certain polymers having a hydrophilic substituent on the polymer backbone have surfactant activity, for example, povidone, polyvinyl alcohol, and glycol ether-based compounds. Compounds derived from long chain fatty acids are a third substantial group of emulsifying and suspending agents usable in this invention. Though any of the foregoing surfactants can be used so long as they are non-toxic, glycol ether-based surfactants are preferred. Preferred surfactants are non-ionic. These include polyethylene glycols (especially PEG 200, 300, 400, 600 and 900), Span®, Arlacel®, Tween®, Myrj®, Brij® (all available from ICI, America's Inc., Wilmington, Delaware), polyoxyethylene, polyol fatty acid esters, polyoxyethylene ether, polyoxypropylene fatty ethers, bee's wax derivatives containing polyoxyethylene, polyoxyethylene lanolin derivatives, polyoxyethylene fatty glycerides, glycerol fatty acid esters or other polyoxyethylene acid alcohol or ether derivatives of long-chain fatty acids of 12-21 carbon atoms. The presently preferred surfactant is Tween® 80 (otherwise known as polysorbate 80 for polyoxyethylene 20 sorbitan monooleate), although it should be understood that any of the above-mentioned surfactants would be suitable after lack of toxicity is demonstrated.

An "emulsion-stabilizing amount of a glycol ether-based surfactant" is usually effected by having the surfactant present in an amount of 0.05 to 5%. An amount of 0.2% to 1% is preferred.

### PREPARATION

The components of the adjuvant of the invention may be obtained through commercial sources, or may be prepared by one of ordinary skill in the art.

The tetra-polyols may be prepared by the process disclosed in US-A-2,979,528, or may be obtained commercially from BASF-Wyandotte under the trademark Tetronic®.

The POP-POE block polymers can be prepared by the methods set out in US-A-2,674,619 issued to Lunsted, and are commercially available from BASF-Wyandotte under the trademark Pluronic®.

The glycol-ether based surfactants PEG 200, 300, 400, 600 and 900, Span®, Arlacel®, Tween®, Myrj®, Brij®, and the like are readily available commercially from ICI, America's Inc., Wilmington, Delaware, and others.

The non-toxic metabolizable oils are available from a variety of sources: e.g., squalane and squalene are available from Aldrich Chemical Co.

The MDPs may be obtained commercially from sources such as Sigma Chemical Co., or prepared following the processes disclosed in Audibert et al., US-A-4,158,052; Audibert et al., US-A-4,220,637; Audibert et al., US-A-4,323,559; Baschang et al., US-A-4,323,560; Baschang et al., US-A-4,409,209; Baschang et al., US-A-4,423,038; Derrien et al., US-A-4,185,089; Hartmann et al., US-A-4,406,889; Jones et al., US-A-4,082,735; Jones et al., US-A-4,082,736; Le Francier et al., US-A-4,427,659; Le Francier et al., US-A-4,461,761; Yamamura et al., US-A-4,314,998; Yamamura et al., US-A-4,101,536; and Yamamura et al., US-A-4,369,178.

The adjuvant is prepared by emulsification, using a mixer. If the adjuvant is to be prepared on a laboratory scale using a tetra-polyol for immediate use, it may be mixed simply by hand. For example, Tween® 80 and buffered saline are added to squalene and Tetronic® 1501 in a test tube at 2X concentration, and the combination mixed using a vortex mixer to form an emulsion. To this is added a 2X solution of antigen and MDP in buffered saline to form the completed vaccine. It is more preferred to use a high-shear mixer such as a Greerco Homogenizer Mixer to form a smoother, more homogenous emulsion.

Preferably, the adjuvant emulsion is "microfluidized" prior to adding the antigen, whether a tetra-polyol or a POP-POE block polymer is used. This is accomplished using a very high-shear mixer such as a Microfluidizer® (commercially available through Microfluidics Corp., Newton, MA). With the Microfluidizer®, typically 100-500 mL batches of emulsion are prepared. The emulsion is cycled through the Microfluidizer® about 2-10 times, until the emulsion particle size reaches the desired level, preferably a diameter less than 800 nm, preferably less than about 300 nm, most preferably, less than about 200 nm. The Microfluidizer® combines shear, turbulence and cavitation forces, the two fluidized streams interacting at very high velocities within an interaction chamber thus creating uniformly small emulsion particles. It should be understood that equipment other than the Microfluidizer® may be capable of producing a satisfactory emulsion, and that the use of any device capable of producing an emulsion of sufficient stability and sufficiently small particle size is within the scope of this process. Normally, the MDP will be added after the emulsification step. If the emulsion is to be stored prior to the addition of the MDP and the antigen, this may be done with refrigeration and/or under nitrogen.

The resulting emulsion may be assayed in a variety of ways well known in the art. The emulsion stability may be measured by allowing the emulsion to stand at room temperature, and under refrigeration, followed by observation for separation into phases. This assay may be accelerated by centrifuging the emulsion, e.g., for two hours at 4500g.

Particle size may be determined by, for example, optical microscopy, transmission electron microscopy, and laser light-scattering techniques, preferably using laser photon correlation spectroscopy (PCS). PCS analysis may be performed using, for example, a Nicomp Model 200 laser particle sizer, with a model TC-100 computing autocorrelator.

Biological activity may be assayed using standard laboratory techniques, e.g., by vaccinating a standard laboratory animal (e.g., a Guinea pig) with a standard antigen (e.g., BSA or DNP-BSA) using a test adjuvant formulation. After allowance of time for boosting the vaccination, and time for immunization to occur, the animal is challenged with the standard antigen and the results measured. The response may be quantified by any measure accepted in the art for measuring immune responses, e.g., in terms of serum antibody titer against the standard antigen (for humoral immunity) and skin test reaction (for cell-mediated immunity).

### ADMINISTRATION

It will be apparent to one of ordinary skill in the art that the precise amounts of MDP derivative and antigen needed to produce a given effect will vary with the particular compounds and antigens, and with the size, age, and condition of the subject to be treated. Thus, it is impossible to state exactly the amounts needed: however, these amounts can easily be determined using methods known to those of ordinary skill in the art.

The adjuvants and vaccines of the invention are generally administered by injection, particularly intramuscular injection, preferably into a large muscle.

In general, an initial vaccination is administered using the desired antigen and the formulation of the invention. The vaccination is "boosted" several weeks later (usually 2-6 weeks, for example, 4-6 weeks) using a vaccine of the invention with or without (preferably with) the MDP component. Generally, 1-2 mL of a vaccine (such as are described in the Examples below) is administered to a human subject in the pratice of the invention.

The following examples are presented as an aid to those of ordinary skill in the art, and are not to be considered as a limitation of the invention in any way.

### EXAMPLE 1

### (Immunogenicity)

### (A) Preparation of Formulations:

Adjuvant formulations were prepared as follows for assay of biological activity. Each emulsion was prepared at 2X concentration prior to combination with a 2X solution of antigen.
Formulation 1 (from Allison, US-A-4,606,918): 5.0% Pluronic® L121, 10% squalane, 0.4% Tween® 80, qs phosphate buffered saline (pH 7.4); the components were added to a test tube and vortex-mixed until a milky emulsion was obtained. This formulation was prepared immediately prior to administration.
Formulation 2 (Formulation 1 with refrigeration): 5.0% Pluronic® L121, 10% squalane, 0,4% Tween® 80, qs phosphate buffered saline (pH 7.4); the components were added to a test tube and vortex-mixed until a milky emulsion was obtained. The formulation was then refrigerated at 4°C beginning one day prior to administration.
Formulation 3 (tetra-polyol formulation of the invention): 5.0% Tetronic® 1501, 10% squalane, 0.4% Tween® 80, qs phosphate buffered saline (pH 7.4); the components were added to a test tube and vortex-mixed until a milky emulsion was obtained.
Formulation 4 (microfluidized POP-POE adjuvant of the invention): 5.0% Pluronic® L121, 10% squalane, 0.4% Tween® 80, qs phosphate buffered saline (pH 7.4); the components were added to a test tube and vortex-mixed until a milky emulsion was obtained. This emulsion was then passed through a Microfluidizer® four times. This formulation was refrigerated with Formulation 2.

To each formulation was then added solid N-acetylmuramyl-L-threonyl-D-isoglutamine (Thr-MDP) to a concentration of 500 µg/mL, to form the complete adjuvant "concentrate." The concentrate was then mixed with a 2X concentration solution of antigen (ovalbumin in saline, 1 mg/mL) to form a test vaccine.

### (B) Bioactivity:

Each test vaccine (0.2 mL) was administered to 8 female Guinea pigs. At four weeks following administration, each animal was boosted with the same test vaccine (but without the Thr-MDP). Antibody titer was measured for serum samples collected at weeks 4 and 6 after initial administration. At 6 weeks, each animal received ovalbumin intradermally, and the diameter of the erythema, and the infiltration rating were determined after 24 hours, as an indication of cell-mediated immunity.

The results are reported in Tables 1 and 2. Each entry represents the mean obtained from 8 animals. Antibody titers were determined by hemagglutination. Infiltration was scored visually, on a 1-3 scale (1 being the weakest response, and 3 being a very obvious swelling at the skin test site).

**TABLE 1**

| Antibody Titer Results | | |
|---|---|---|
| Formulation | 4 weeks | 6 weeks |
| 1 (control POP-POE) | 2.25 ± .38 | 6.13 ± .30 |
| 2 (refrigerated POP-POE) | 1.38 ± .18 | 6.50 ± .19 |
| 3 (tetra-polyol) | 4.00 ± .38 | 8.13 ± .30 |
| 4 (refrig/microfluidized POP-POE) | 2.87 ± .12 | 8.00 ± .27 |

**TABLE 2**

| Cell-Mediated Immunity Results | | |
|---|---|---|
| Formulation | Diameter (mm) | Infiltration |
| 1 (control POP-POE) | 16.06 | 1.75 |
| 2 (refrigerated POP-POE) | 13.81 | 1.31 |
| 3 (tetra-polyol) | 19.13 | 1.94 |
| 4 (refrig/microfluidized POP-POE) | 13.56 | 1.50 |

The results demonstrate that the tetra-polyol adjuvants are significantly more effective for increasing the immunogenicity of antigens, and that the microfluidized POP-POE block polymer adjuvants are at least as effective as control formulations while demonstrating superior storage stability.

### EXAMPLE 2

### (Physical Characteristics)

The formulations prepared in Example 1(A) were examined for physical characteristics.
(A) Separation: Each of the formulations (1-4) was centrifuged for 30 minutes at 4500 x G, then allowed to stand to separate into layers. The amount of separation that occurred was noted and estimated as the volume percentage of the total occupied by the upper layer. Formulations 1-3 separated about 10%, while Formulation 4 separated less than about 1%.
(B) Particle Size: Particle size distributions were analyzed by optical microscopy (Leitz Ortholux II POL-BK polarized light microscope), transmission electron microscopy (TEM, using a Hitachi model HS-8-1), and laser photon correlation spectroscopy (PCS, using a Nicomp Model 200 laser particle sizer, with a model TC-100 computing autocorrelator). Particle size distributions were determined for top layers and bottom layers separately. Samples analyzed by TEM and PCS were diluted 1:100 or greater before analysis. The results demonstrated that Formulations 1-3 exhibited particle sizes ranging from < 0.1 µm to about 25 µm. Formulatio 4 exhibited particle sizes ranging from < 0.1 µm to about 0.3 µm (300 nm).

### EXAMPLE 3

### (Formulations)

Exemplary adjuvant formulations were prepared as follows:
(A) Tetronic®/Thr-MDP:

| | |
|---|---|
| Tetronic® 1501 | 2.5 g |
| Squalane | 5.0 g |
| Tween® 80 | 0.2 g |
| Thr-MDP | 250.0 mg |
| Phosphate buffered saline | qs to 100.0 mL |

The Tetronic® 1501, squalane, and Tween® 80 are placed in an appropriate vessel with 85 mL of phosphate buffered saline (PBS) and are mixed with a mechanical mixer (Greerco Homogenizer-Mixer, model #1L-79, Greerco Corp., Hudson, New Hampshire) at about 4750 rpm for about 30-60 minutes. Then, the Thr-MDP (N-acetylmuramyl-L-threonyl-D-isoglutamine) and remaining 15 mL of PBS are stirred in, producing an adjuvant formulation of the invention.
(B) Similarly, proceeding as in part (A) above but substituting N-acetylmuramyl-L-α-aminobutyryl-D-isoglutamine (Abu-MDP), 6-O-stearoyl-N-acetylmuramyl-L-α-aminobutyryl-D-isoglutamine (Abu-MDP stearate), N-acetylmuramyl-L-valyl-D-isoglutamine (Val-MDP), N-acetylmuramyl-L-alanyl-D-isoglutamine (MDP), N-acetyldesmethylmuramyl-L-alanyl-D-isoglutamine (desMe-MDP), N-acetylmuramyl-L-alanyl-D-glutamine butyl ester (murabutide), n-butyrylmuramyl-L-(α-aminobutyryl)-D-isoglutamine, and N-acetylmuramyl-L-seryl-D-isoglutamine (Ser-MDP), for the Thr-MDP, the corresponding adjuvant formulations are prepared.
(C) Pluronic®/Thr-MDP:

| | |
|---|---|
| Pluronic® L121 | 2.5 g |
| Squalane | 5.0 g |
| Tween® 80 | 0.2 g |
| Thr-MDP | 250.0 mg |
| Phosphate buffered saline | qs to 100.0 mL |

The Pluronic® L121, squalane, and Tween® 80 are placed in an appropriate vessel with 85 mL of phosphate buffered saline (PBS) and are mixed with a mechanical mixer (e.g., Greerco Homogenizer-Mixer) at about 4750 rpm for about 5-10 minutes. Then, the Thr-MDP (N-acetylmuramyl-L-threonyl-D-isoglutamine) and remaining 15 mL of PBS are stirred in. The resulting emulsion is then processed through a Microfluidizer® (Microfluidics Corp.) for at least 4 cycles to provide an adjuvant formulation of the invention. Alternatively, the MDP is added after the microfluidization step.
(D) Similarly, proceeding as in part (C) above but substituting N-acetylmuramyl-L-α-aminobutyryl-D-isoglutamine (Abu-MDP), 6-O-stearoyl-N-acetylmuramyl-L-α-aminobutyryl-D-isoglutamine (Abu-MDP stearate), N-acetylmuramyl-L-valyl-D-isoglutamine (Val-MDP), N-acetylmuramyl-L-alanyl-D-isoglutamine (MDP), N-acetyl-desmethylmuramyl-L-alanyl-D-isoglutamine (desMe-MDP), N-acetylmuramyl-L-alanyl-D-glutamine butyl ester (murabutide), N-butyrylmuramyl-L-(α-aminobutyryl)-D-isoglutamine, and N-acetylmuramyl-L-seryl-D-isoglutamine (Ser-MDP), for the Thr-MDP, the corresponding adjuvant formulations are prepared.
(E) Similarly, the adjuvant formulations prepared in parts A and B above may be further improved by microfluidizing as described in parts C and D to form the corresponding microfluidized Tetronic® formulations.
(F) Tetronic®/murabutide:

| | |
|---|---|
| Tetronic® 1501 | 2.0 g |
| Squalane | 6.0 g |
| Brij® 80 | 0.3 g |
| murabutide | 300.0 mg |
| Phosphate buffered saline | qs to 100.0 mL |

The formulation is prepared as described in parts A and E above.
(G) Similarly, proceeding as described in parts A-F above, but substituting Pluronic® L101 or Tetronic® 1301 for Pluronic® L121 or Tetronic® 1501, the corresponding adjuvants are prepared.
(H) Pluronic®/Ser-MDP concentrate:

| | |
|---|---|
| Pluronic® L121 | 2.5 g |
| Squalane | 5.0 g |
| Tween® 80 | 0.2 g |
| Ser-MDP | 250.0 mg |
| Phosphate buffered saline | qs to 100.0 mL |

The Pluronic® L121, squalene, and Tween® 80 are placed in an appropriate vessel with PBS (qs to 50 mL) and are mixed with a mechanical mixer (Greerco Homogenizer-Mixer) at about 4750 rpm for about 5-10 minutes. The resulting emulsion is then processed through a Microfluidizier® for 4 to 10 cycles to provide an emulsion concentrate. The Ser-MDP and remaining PBS are provided as a second component to form a two-component "kit" for extemporaneous preparation of the adjuvant. To use, the desired amount of antigen is added to the Ser-MDP solution, and the resulting solution is mixed vigorously with the emulsion component.
(I) Similarly, proceeding as in part H above but substituting the components described in parts A-G above, the corresponding adjuvant kits are prepared.
(J) Vaccines: Vaccines of the invention are prepared by adding an appropriate amount of antigen to any of the formulations described above. Suitable antigens include antigens for hepatitis B, influenza (for example, A or B), AIDS and herpes. The vaccine may contain more than one antigen if desired, for example, antigens for diphtheria, pertussis, and tuberculosis may be coadministered in a single formulation.

For ease of preparation, a small portion of the PBS used may be withheld from the adjuvant preparation, e.g., one may prepare the adjuvants described above using 90 mL rather than 100 mL, and use the withheld PBS to dissolve/suspend the antigen(s). The antigen/PBS solution is then mixed with the (slightly) concentrated emulsion to prepare the final vaccine. Alternatively, and more preferably, an adjuvant emulsion (without MDP) of two times concentration is mixed with an antigen/MDP solution of two times concentration.

### EXAMPLE 4

### Comparison of Freshly Made and Frozen Emulsions

Two times concentrated microfluidized emulsions, consisting of 10% v/v squalane, 5% v/v Pluronic® L121 and 0.4% polysorbate 80 in phosphate buffered saline, were used in the test, having been prepared as for Formulation 4 (Example 1). One emulsion was stored frozen for seven days before use, while the other was freshly prepared and kept at room temperature. On the day vaccines were prepared, Thr-MDP was added to the fresh and thawed emulsions. Equal volumes of 2X concentrated ovalbumin were added to the 2 lots of emulsions just before the vaccines were used to immunize groups of 8 female guinea pigs. The final concentrations of the constituents of the vaccines were: Phosphate buffered saline 92.33%; Squalane 5%; Pluronic® L121, 2.5%; Polysorbate 80, 0.17%; Thr-MDP 250 µg/ml; and Ovalbumin 1.0 mg/ml.

Guinea pigs were vaccinated on days 0 and 28 with 0.2 ml of vaccine per animal, bled on days 28 and 42, and skin tested with 10 µg of ovalbumin on day 42.

The results obtained, as shown below, show that the efficacy of the frozen material was equivalent to that of the freshly prepared emulsion.

**TABLE 1**

| ANTIBODY TITRES^{a} | | | | | |
|---|---|---|---|---|---|
| Group | No. of Animals | Vehicle Preparation | 28 Days | 42 Days | |
| | | | Mean Titre ± SE | Mean Titre ± SE | Equivalent Dilution^{b} |
| 1 | 8 | Fresh | 4.6 ± 0.2 | 9.0 ± 0.1 | 18,837 |
| 2 | 8 | Frozen | 5.1 ± 0.3 | 8.9 ± 0.1 | 17,830 |

| | | | | | |
|---|---|---|---|---|---|
| ^{a} Titres are expressed as log₃ of the reciprocal of the serum dilution giving an optical density reading of 0.5 absorbance units, under the conditions of the assay. | | | | | |
| ^{b} Titre expressed as the reciprocal of the mean serum dilution. | | | | | |

**TABLE 2**

| DELAYED HYPERSENSITIVITY SKIN REACTIONS | | | | |
|---|---|---|---|---|
| Group | No. of Animals | Vehicle Preparation | Mean Diameter (mm ± SE) | |
| | | | 24 Hr | 48 Hr |
| 1 | 8 | Fresh | 14.3 ± 0.7 | 11.0 ± 1.7 |
| 2 | 8 | Frozen | 13.3 ± 2.3^{a} | 11.4 ± 2.9^{a} |

| | | | | |
|---|---|---|---|---|
| ^{a} Includes one animal which had no response and may not have been skin tested. | | | | |

### EXAMPLE 5

### Hepatitis Virus Vaccine

Groups of 8 female Hartley guinea pigs were immunized subcutaneously with a vaccine consisting of 0.5 µg or 0.1 µg of Hepatitis B virus surface antigen (HBsAg) in microfluidized adjuvant (prepared as for Formulation 4, Example 1, without refrigeration) or adsorbed to alum (commercially available hepatitis vaccine). The HBsAg in saline and HBsAG adsorbed to alum were provided by Merck Sharpe and Dohme Research Laboratories. The adjuvant preparation consisted of 92.33% PBS, 5% squalane, 2.5% Pluronic L121, 0.17% polysorbate 80, 100 µg/ml Thr-MDP, and either 1.0 µg/ml or 0.2 µg/ml of HBsAg. Each animal received 0.5 ml of vaccine at day 0 and week 4. The animals were bled at weeks 4, 6 and 15. Antibody titres were determined by ELISA techniques and were far superior for the vaccine of the invention.

**TABLE**

| ANTI-HBsAG TITRES OF POOLED GUINEA PIG SERA AT 4, 6 OR 15 WEEKS AFTER PRIMARY VACCINATION | | | | | |
|---|---|---|---|---|---|
| Group | Vehicle | HBsAg Dose (µg) | ELISA Titre | | |
| | | | 4 Weeks | 6 Weeks | 15 Weeks |
| 1 | Adjuvant | 0.5 | 814 | 34092 | 16693 |
| 2 | Alum | 0.5 | 230 | 4002 | 3041 |
| 3 | Adjuvant | 0.1 | 52 | 6210 | 4719 |
| 4 | Alum | 0.1 | 34 | 1131 | 1409 |

### EXAMPLE 6

### Influenza Virus Vaccine

### MATERIALS AND METHODS

Groups of 10 or 11 6-7 week old female BALB/cJ mice were immunized subcutaneously with the trivalent vaccine prepared for the 1987-88 influenza season. The influenza virus strains used were A/Taiwan, A/Leningrad and B/Ann Arbor. The antigen concentration is expressed in µg of hemagglutinin (HA). The vaccine was diluted so that the mice received 0.01 µg of HA of each strain in 0.1 ml of a microfluidized adjuvant of the invention (2.5% Pluronic® L121; 5.0% Squalane; 0.17% polysorbate 80; 500 µg/ml Thr-MDP; (0.1 µg/ml influenza;) 92.33% PBS; prepared as for Formulation 4, Example 1, without refrigeration). One group of mice was given adjuvant (with Thr-MDP) without any influenza antigen. The groups of mice were immunized as follows:
1. Control - Adjuvant only
2. 0.01 µg of each strain in Adjuvant
3. 0.01 µg of each strain in Adjuvant
Groups 1 and 2 were immunized at 0 and 3 weeks while group 3 was immunized at 0 time only.

At weeks 3, 5 and 9, 50 µl of blood was obtained from each mouse (under ether anesthesia) via the retro-orbital plexus. Sera were pooled by group. At week 13 the mice were bled out under ether anesthesia, and sera were kept individually as well as in pools.

The Table below shows the mean titres determined for sera from all groups at week 13. For the 0.01 µg dose levels, there was no significant difference in titre between the groups given one dose compared to those given 2 doses, when the anti-A/Taiwan or anti-A/Leningrad titres were measured. However, one dose induced significantly lower anti-B/Ann Arbor titres than did 2 doses. (Compare groups 2 and 3).

**TABLE**

| MEAN ANTI-HA TITRES OF MICE 13 WEEKS FOLLOWING IMMUNIZATION | | | | | | |
|---|---|---|---|---|---|---|
| Group | HA Dose (µg) | Boost at 3 wks | Vehicle | Mean Titre ± SE | | |
| | | | | A/Taiwan | A/Leningrad | B/Ann Arbor |
| 1 | 0 | + | Adjuvant | <3.0 ± 0^{b}^{,}^{c} | <3.0 ± 0^{b} | <3.0 ± 0^{b} |
| 2 | 0.01 | + | Adjuvant | 9.1 ± 0.1 | 8.0 ± 0.2 | 6.8 ± 0.2 |
| 3 | 0.01 | - | Adjuvant | 8.8 ± 0.2 | 7.8 ± 0.3 | 5.9 ± 0.3 |

| | | | | | | |
|---|---|---|---|---|---|---|
| ^{a} Titre is log₃ of the reciprocal of the serum dilution giving an optical density of 0.5 absorbance units. | | | | | | |
| ^{b} Lowest dilution tested was 1/27, i.e., 1/3³ | | | | | | |
| ^{c} Sera of 2 animals had titres of 3.1 and 3.2, while for the remaining B sera no antibody was detectable. | | | | | | |

### EXAMPLE 7

The ovalbumin vaccine of Example 4 was prepared as described in Formulation 4, Example 1, but without the Tween® 80.

### EXAMPLE 8

The ovalbumin vaccine of Example 4 was prepared as described in Formulation 4, Example 1, but the vaccine composition contained only 1.25% Pluronics® L121.

### EXAMPLE 9

### Other Vaccines

The ovalbumin vaccine of Example 4 was prepared as described in Formulation 4, Example 1, but using the following antigens in place of ovalbumin:
HIV (Human immunodeficiency virus)
Plasmodium yoelii peptides
Influenza viruses (A and B types)
Adenoviruses
Herpes simplex virus type 1, glycoprotein gD1
Melanoma antigens (mouse and human)
Foot and mouth disease virus
Hepatitis B virus surface antigens
Hepatitis A virus
Para-influenza 3 glycoproteins
SIV (simian immunodeficiency virus)
Shistoma mansoni cercaria
Folate hydrolase
Polio virus
Mouse idiotype antibody
Bacterial toxoids
Human tumor associated antigens
Simian retrovirus (type 1 & 2) peptides
Type D retrovirus
Parasite antigens
LHRH
Mouse IgG peptides
Brucella abortus proteins
HIV proteins
Fibroblast growth factors (α and β)
IL-6
Herpes simplex virus, type 2, early gene 22
Feline leukemia virus
[The weight of MDP in the vaccine was subject to minor variation depending on the species of animal tested. In some cases the emulsion was not refrigerated before addition of the antigen and MDP.]

### EXAMPLE 10

An ovalbumin vaccine in microfluidized adjuvant consisting of:
2.5% Tetronic® 1501
5% Squalane
0.2% Tween® 80
Phosphate buffered saline to volume
250 µg/ml Thr-MDP
Ovalbumin
was prepared as for Formulation 4, Example 1, without refrigeration.

A group of 8 female Sim:(HA) guinea pigs, 350-400 g, was used. The animals were injected sc in the nuchal region with 0.2 ml of the formulation; each animal received 200 µg OA and 50 µg Thr-MDP on Day 0, and 50 µg OA and 50 µg Thr-MDP on Day 28. They were bled by cardiac puncture on Days 28 and 42, and skin tested on Day 42 with 10 µg OA, given id. The diameter and induration of the skin tests were measured 24 and 42 hours later. The antibody titres were determined by passive hemagglutination and by ELISA (see below).

| ANTIBODY TITRES - ELISA METHOD | | |
|---|---|---|
| 28 Days | 42 Days | Actual Dilution |
| Titre ± SE | Titre ± SE | |
| 5.26 ± 0.25 | 9.46 ± 0.10 | 32,626 |

| DELAYED HYPERSENSITIVITY | | | | | |
|---|---|---|---|---|---|
| Mean Diameter ± SE | | No. Inf. | | Inf. Score | |
| 24 Hr | 48 Hr | 24 Hr | 48 Hr | 24 Hr | 48 Hr |
| 16.78 ± 0.64 | 12.00 ± 0.49 | 5/7 | 4/7 | 1.50 | 1.36 |

### EXAMPLE 11

In the tests reported above, no significant side effects were observed.

## Claims (Claims for the following Contracting State(s): AT, BE, CH, DE, FR, GB, IT, LI, LU, NL, SE)

1. An adjuvant in the form of an emulsion having oily particles dispersed in a continuous aqueous phase, for potentiating the immunogenicity of an antigen, which adjuvant comprises:
an emulsion-forming amount of a non-toxic N,N,N',N'-tetra(polyoxyproprlene-polyoxyethylene)-1,2-diaminoethane block polymer ("tetra-polyol") or of a polyoxypropylene-polyoxyethylene ("POP-POE") block polymer; and
an immunopotentiating amount of a glycopeptide;
wherein substantially all of said oily particles have a diameter less than about 800 nm.

2. An adjuvant according to Claim 1 in the form of an emulsion having oily particles dispersed in a continuous aqueous phase, for potentiating the immunogenicity of an antigen, which adjuvant comprises:
an emulsion-forming amount of a non-toxic tetra-polyol;
optionally, an emulsion-forming amount of a non-toxic metabolizable oil;
an emulsion-stabi1izing amount of a glycol ether-based surfactant;
water or aqueous solution; and
an immunopotentiating amount of a muramyldipeptide derivative of formula I 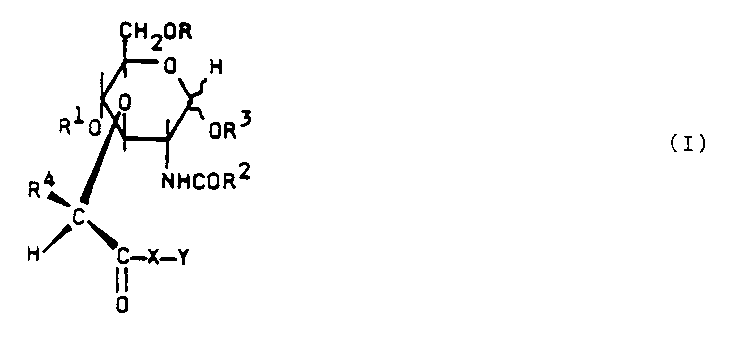 and the pharmaceutically acceptable salts thereof, wherein
R and R₁ are each independently H or acyl of 1 to 22 carbon atoms;
R₂ is alkyl or aryl, optionally substituted with halo, nitro, or lower alkyl;
R₃ is H, alkyl, or aryl;
R₄ is H or lower alkyl;
X is L-alanyl, L-α-aminobutyryl, L-arginyl, L-asparginyl, L-aspartyl, L-cysteinyl, L-glutaminyl, L-glutamyl, glycyl, L-histidyl, L-hydroxyprolyl, L-isoleucyl, L-leucyl, L-lysyl, L-methionyl, L-ornithinyl, Lphenylalanyl, L-prolyl, L-seryl, L-threonyl, L-tyrosyl, L-tryptophanyl, or L-valyl; and
Y is D-glutamine, D-isoglutamine or D-isoasparagine.

3. The adjuvant of Claim 1 or 2 wherein said tetra-polyol is Tetronic® 1501 which has an average molecular weight of the POP section of 6500-7000, and a percentage of POE of 10%.

4. The adjuvant of Claim 3 which includes a non-toxic metabolizable oil, wherein said oil is squalene or squalane.

5. The adjuvant of Claim 3 or 4 wherein said glycol ether-based surfactant is polysorbate 80.

6. The adjuvant of Claim 3, 4 or 5 wherein said water or aqueous solution comprises isotonic buffered saline.

7. The adjuvant of any one of Claims 1-6 wherein substantially all of said oily particles have a diameter less than about 300 nm.

8. The adjuvant of any one of Claims 2-7 wherein said muramyl- dipeptide derivative of formula I is:
N-acetylmuramyl-L-threonyl-D-isoglutamine,
N-acetylmuramyl-L-α-aminobutyryl-D-isoglutamine,
6-O-stearoyl-N-acetylmuramyl-L-α-aminobutyryl-D-isoglutamine,
N-acetylmuramyl-L-valyl-D-isoglutamine,
N-acetylmuramyl-L-alanyl-D-isoglutamine,
N-acetyl-desmethylmuramyl-L-alanyl-D-isoglutamine,
N-acetylmuramyl-L-alanyl-D-glutamine butyl ester,
N-acetylmuramyl-L-seryl-D-isoglutamine, or
N-butyrylmuramyl-L-α-aminobutyryl-D-isoglutamine.

9. An adjuvant according to any one of the preceding claims in the form of an oil-in-water type emulsion, having oily particles dispersed in a continuous aqueous phase, for potentiating the immunogenicity of an antigen, which adjuvant comprises:
a non-toxic tetra-polyol in an amount of 0.2 to 49%;
a non-toxic metabolizable oil in an amount of 0-15%;
a glycol ether-based surfactant in an amount of 0.05-5%;
water or aqueous solution; and
0.0001-10% a muramyldipeptide derivative of formula I.

10. An adjuvant according to Claim 9 in the form of an oil-in-water type emulsion, having oily particles dispersed in a continuous aqueous phase, for potentiating the immunogenicity of an antigen, which adjuvant comprises:
Tetronic® 1501 in an amount of 1-10%;
squalane or squalene in an amount of 1-10%;
polysorbate 80 in an amount of about 0.2%;
isotonic buffered saline; and
0.0001-10% N-acetylmuramyl-L-threonyl-D-isoglutamine.

11. An adjuvant according to Claim 1 in the form of an oil-in-water type emulsion, having oily particles dispersed in a continuous aqueous phase, for potentiating the immunogenicity of an antigen, which adjuvant comprises:
an emulsion-forming amount of a non-toxic POP-POE block polymer;
optionally, an emulsion-forming amount of a non-toxic metabolizable oil;
an emulsion-stabilizing amount of a glycol ether-based surfactant;
water or aqueous solution; and
an immunopotentiating amount of a muramyldipeptide derivative of formula I 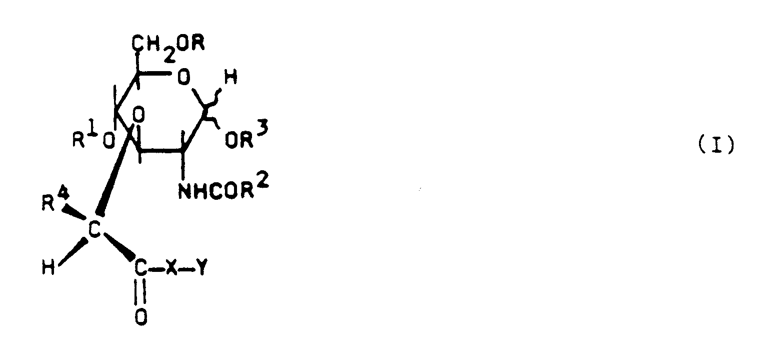 and the pharmaceutically acceptable salts thereof, wherein
R and R₁ are each independently H or acyl of 1 to 22 carbon atoms;
R₂ is alkyl or aryl, optionally substituted with halo, nitro, or lower alkyl;
R₃ is H, alkyl, or aryl;
R₄ is H or lower alkyl;
X is L-alanyl, L-α-aminobutyryl, L-arginyl, L-asparginyl, L-aspartyl, L-cysteinyl, L-glutaminyl, L-glutamyl, glycyl, L-histidyl, L-hydroxyprolyl, L-isoleucyl, L-leucyl, L-lysyl, L-methionyl, L-ornithinyl, L-phenylalanyl, L-prolyl, L-seryl, L-threonyl, L-tyrosyl, L-tryptophanyl, or L-valyl; and
Y is D-glutamine, D-isoglutamine or
D-isoasparagine,
wherein substantially all of said oily particles have a diameter less than about 800 nm, preferably less than about 300 nm.

12. The adjuvant of Claim 11 wherein said non-toxic POP-POE block polymer is Pluronic® L121 wherein the average molecular weight of the POP sect ion is 4000, and the percentage of POE is 10%.

13. The adjuvant of Claim 11 or 12 wherein said muramyl- dipeptide derivative of formula I is N-acetylmuramyl-L- threonyl-D-isoglutamine.

14. The adjuvant of any one of Claims 11-13 which includes a non-toxic metabolizable oil, wherein said oil is squalene or squalane.

15. The adjuvant of any one of Claims 11-14 wherein said glycol ether-based surfactant is polysorbate 80.

16. The adjuvant of any one of Claims 11-15 wherein said water or aqueous solution comprises isotonic buffered saline.

17. An adjuvant according to Claim 11 in the form of an oil-in-water type emulsion, having oily particles dispersed in a continuous aqueous phase, for potentiating the immunogenicity of an antigen, which adjuvant comprises:
a non-toxic POP-POE block polymer in an amount of 0.2 to 49%;
a non-toxic metabolizable oil in an amount of 0-15%;
a glycol ether-based surfactant in an amount of 0.05-5%;
water or aqueous solution; and
0.0001-10% a muramyldipeptide derivative of formula I
wherein substantially all of said oily particles have a diameter less than about 800 nm.

18. An adjuvant according to Claim 17 in the form of an oil-in-water type emulsion, having oily particles dispersed in a continuous aqueous phase, for potentiating the immunogenicity of an antigen, which adjuvant comprises:
Pluronic® L121 in an amount of 1-10%;
squalane or squalene in an amount of 1-10%;
polysorbate 80 in an amount of about 0.2%;
isotonic buffered saline; and
0.0001-10% N-acetylmuramyl-L-threonyl-D-isoglutamine, wherein substantially all of said oily particles have a diameter less than about 800 nm.

19. A vaccine in the form of an oil-in-water type emulsion, having oily particles dispersed in a continuous aqueous phase, for immunizing an animal, which vaccine comprises:
an immunogenic amount of an antigen; and
an adjuvant according to any one of the Claims 1-18.

20. A process for preparing the adjuvant according to any one of the claims 1-18,
which process comprises mixing together the aqueous phase and the emulsion-forming amount of the non-toxic tetra-polyol or of the POP-POE block polymer so as to form an emulsion, wherein substantially all oily particles have a diameter less than about 800 nm.

21. A process according to Claim 20 wherein the glycopeptide is added to the mixture after the emulsion is formed.

22. A process for preparing the vaccine according to Claim 19, which process comprises mixing the antigen with the adjuvant.

23. A kit for extemporaneous preparation of an adjuvant according to any one of Claims 1-18, which kit comprises:
a first container containing the emulsion of the tetra-polyol or POP-POE polymer in the aqueous phase where substantially all the oily particles have a diameter less than about 800 nm; and
a second container containing the glycopeptide.

24. A kit according to Claim 23 for extemporaneous preparation of an adjuvant of the invention, which kit comprises:
a first container containing an oil-in-water type emulsion, having oily particles dispersed in a continuous aqueous phase, where said emulsion comprises Tetronic® 1501 or Pluronic® L121, squalane or squalene, polysorbate 80, and isotonic buffered saline, where substantially all of said oily particles have a diameter less than about 800 nm; and
a second container containing N-acetylmuramyl-L-threonyl-D-isoglutamine;
where the concentrations of the components in each container are selected such that combination of the contents of both containers produces a formulation comprising Tetronic® 1501 or Pluronic® L121 in an amount of 1-30%, squalane or squalene in an amount of 1-30%, polysorbate 80 in an amount of about 0.2-5%, 0.0001-30% N-acetylmuramyl-L-threonyl-D-isoglutamine, and isotonic buffered saline.

25. A kit for extemporaneous preparation of a vaccine according to Claim 19, which kit comprises:
a first container containing the emulsion of the tetra-polyol or POP-POE block polymer in the aqueous phase where substantially all the oily particles have a diameter less than about 800 nm; and
a second container containing the antigen;
wherein the glycopeptide may be present in a third container, or in the first or second containers.

26. A kit according to Claim 25 for extemporaneous preparation of a vaccine of the invention, which kit comprises:
a first container containing an oil-in-water type emulsion, having oily particles dispersed in a continuous aqueous phase, where said emulsion comprises Tetronic® 1501 or Pluronic® L121, squalane or squalene, polysorbate 80, and isotonic buffered saline, where substantially all of said oily particles have a diameter less than about 800 nm; and
a second container containing N-acetylmuramyl-L-threonyl-D-isoglutamine and an immunogenic amount of an antigen;
where the concentrations of the components in each container are selected such that combination of the contents of both containers produces a formulation comprising Tetronic® 1501 or Pluronic® L121 in an amount of 1-10%, squalane or squalene in an amount of 1-10%, polysorbate 80 in an amount of about 0.2%, 0.0001-10% N-acetylmuramyl-L-threonyl-D-isoglutamine, an immunogenic amount of an antigen, and isotonic buffered saline.

## Claims (Claims for the following Contracting State(s): ES, GR)

1. A process for preparing an adjuvant in the form of an emulsion having oily particles dispersed in a continuous aqueous phase, for potentiating the immunogenicity of an antigen, which adjuvant comprises:
an emulsion-forming amount of non-toxic N,N,N',N'-tetra(polyoxypropylene-polyoxyethylene)-1,2-diaminoethane block polymer ("tetra-polyol") or of a polyoxypropylene-polyoxyethylene ("POP-POE") block polymer; and
an immunopotentiating amount of a glycopeptide;
wherein substantially all of said oily particles have a diameter less than about 800 nm, which process comprises mixing together the aqueous phase and the tetra-polyol or POP-POE block polymer.

2. A process according to Claim 1, for the preparation of an adjuvant in the form of an emulsion having oily particles dispersed in a continuous aqueous phase, for potentiating the immunogenicity of an antigen, which adjuvant comprises:
an emulsion-forming amount of a non-toxic tetra-polyol;
optionally, an emulsion-forming amount of a non-toxic metabolizable oil;
an emulsion-stabilizing amount of a glycol ether-based surfactant;
water or aqueous solution; and
an immunopotentiating amount of a muramyldipeptide derivative of formula I 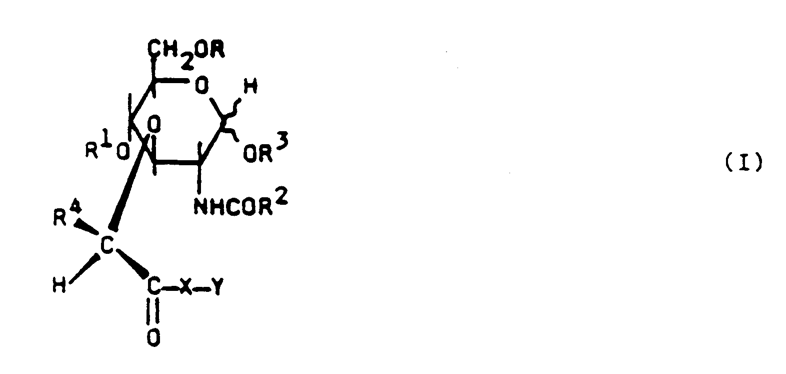 and the pharmaceutically acceptable salts thereof, wherein
R and R₁ are each independently H or acyl of 1 to 22 carbon atoms;
R₂ is alkyl or aryl, optionally substituted with halo, nitro, or lower alkyl;
R₃ is H, alkyl, or aryl;
R₄ is H or lower alkyl;
X is L-alanyl, L-α-aminobutyryl, L-arginyl, L-asparginyl, L-aspartyl, L-cysteinyl, L-glutaminyl, L-glutamyl, glycyl, L-histidyl, L-hydroxyprolyl, L-isoleucyl, L-leucyl, L-lysyl, L-methionyl, L-ornithinyl, L-phenylalanyl, L-prolyl, L-seryl, L-threonyl, L-tyrosyl, L-tryptophanyl, or L-valyl; and
Y is D-glutamine, D-isoglutamine or D-isoasparagine.

3. The process of Claim 1 or 2 wherein said tetra-polyol is Tetronic® 1501 which has an average molecular weight of the POP section of 6500-7000, and a percentage of POE of 10%.

4. The process of Claim 3 which includes mixing a non-toxic metabolizable oil, wherein said oil is squalene or squalane.

5. The process of Claim 3 or 4 wherein said glycol ether-based surfactant is polysorbate 80.

6. The process of Claim 3, 4 or 5 wherein said water or aqueous solution comprises isotonic buffered saline.

7. The process of any one of Claims 1-6 wherein substantially all of said oily particles have a diameter less than about 300 nm.

8. The process of any one of Claims 2-7 wherein said muramyl- dipeptide derivative of formula I is:
N-acetylmuramyl-L-threonyl-D-isoglutamine,
N-acetylmuramyl-L-α-aminobutyryl-D-isoglutamine,
6-O-stearoyl-N-acetylmuramyl-L-α-aminobutyryl-D-isoglutamine,
N-acetylmuramyl-L-valyl-D-isoglutamine,
N-acetylmuramyl-L-alanyl-D-isoglutamine,
N-acetyl-desmethylmuramyl-L-alanyl-D-isoglutamine,
N-acetylmuramyl-L-alanyl-D-glutamine butyl ester,
N-acetylmuramyl-L-seryl-D-isoglutamine, or
N-butyrylmuramyl-L-α-aminobutyryl-D-isoglutamine.

9. A process according to any one of the preceding claims for preparing an adjuvant in the form of an oil-in-water type emulsion, having oily particles dispersed in a continuous aqueous phase, for potentiating the immunogenicity of an antigen, which adjuvant comprises:
a non-toxic tetra-polyol in an amount of 0.2 to 49%;
a non-toxic metabolizable oil in an amount of 0-15%;
a glycol ether-based surfactant in an amount of 0.05-5%;
water or aqueous solution; and
0.0001-10% a muramyldipeptide derivative of formula I.

10. A process according to Claim 9 for preparing an adjuvant in the form of an oil-in-water type emulsion, having oily particles dispersed in a continuous aqueous phase, for potentiating the immunogenicity of an antigen, which adjuvant comprises
Tetronic® 1501 in an amount of 1-10%;
squalane or squalene in an amount of 1-10%;
polysorbate 80 in an amount of about 0.2%;
isotonic buffered saline; and
0.0001-10% N-acetylmuramyl-L-threonyl-D-isoglutamine.

11. A process according to Claim 1 for preparing an adjuvant in the form of an oil-in-water type emulsion, having oily particles dispersed in a continuous aqueous phase, for potentiating the immunogenicity of an antigen, which adjuvant comprises:
an emulsion-forming amount of a non-toxic POP-POE block polymer;
optionally, an emulsion-forming amount of a non-toxic metabolizable oil;
an emulsion-stabilizing amount of a glycol ether-based surfactant;
water or aqueous solution; and
an immunopotentiating amount of a muramyldipeptide derivative of formula I 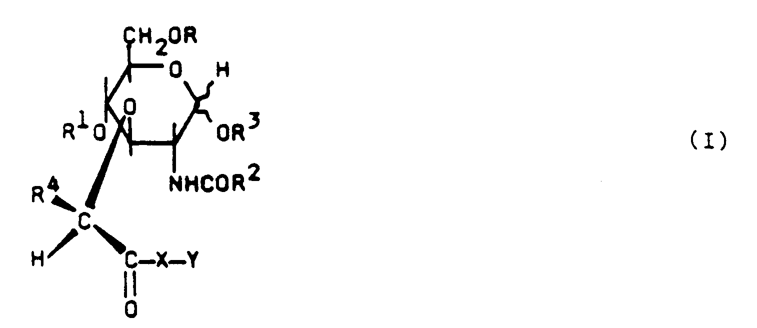 and the pharmaceutically acceptable salts thereof, wherein
R and R₁ are each independently H or acyl of 1 to 22 carbon atoms;
R₂ is alkyl or aryl, optionally substituted with halo, nitro, or lower alkyl;
R₃ is H, alkyl, or aryl;
R₄ is H or lower alkyl;
X is L-alanyl, L-α-aminobutyryl, L-arginyl, L-asparginyl, L-aspartyl, L-cysteinyl, L-glutaminyl, L-glutamyl, glycyl, L-histidyl, L-hydroxyprolyl, L-isoleucyl, L-leucyl, L-lysyl, L-methionyl, L-ornithinyl, L-phenylalanyl, L-prolyl, L-seryl, L-threonyl, L-tyrosyl, L-tryptophanyl, or L-valyl; and
Y is D-glutamine, D-isoglutamine or
D-isoasparagine,
wherein substantially all of said oily particles have a diameter less than about 800 nm, preferably less than about 300 nm.

12. The process of Claim 11 wherein said non-toxic POP-POE block polymer is Pluronic® L121 wherein the average molecular weight of the POP sect ion is 4000, and the percentage of POE is 10%.

13. The process of Claim 11 or 12 wherein said muramyl- dipeptide derivative of formula I is N-acetylmuramyl-L- threonyl-D-isoglutamine.

14. The process of any one of Claims 11-13 which includes mixing a non-toxic metabolizable oil, wherein said oil is squalene or squalane.

15. The process of any one of Claims 11-14 wherein said glycol ether-based surfactant is polysorbate 80.

16. The process of any one of Claims 11-15 wherein said water or aqueous solution comprises isotonic buffered saline.

17. A process according to Claim 11 for preparing an adjuvant in the form of an oil-in-water type emulsion, having oily particles dispersed in a continuous aqueous phase, for potentiating the immunogenicity of an antigen, which adjuvant comprises:
a non-toxic POP-POE block polymer in an amount of 0.2 to 49%;
a non-toxic metabolizable oil in an amount of 0-15%;
a glycol ether-based surfactant in an amount of 0.05-5%;
water or aqueous solution; and
0.0001-10% a muramyldipeptide derivative of formula I
wherein substantially all of said oily particles have a diameter less than about 800 nm.

18. A process according to Claim 17 for preparing an adjuvant in the form of an oil-in-water type emulsion, having oily particles dispersed in a continuous aqueous phase, for potentiating the immunogenicity of an antigen, which adjuvant comprises:
Pluronic® L121 in an amount of 1-10%;
squalane or squalene in an amount of 1-10%;
polysorbate 80 in an amount of about 0.2%;
isotonic buffered saline; and
0.0001-10% N-acetylmuramyl-L-threonyl-D-isoglutamine, wherein substantially all of said oily particles have a diameter less than about 800 nm.

19. A process according to any one of Claims 1-18 wherein the glycopeptide is added to the mixture after the emulsion is formed.

20. A process for preparing a vaccine in the form of an oil-in-water type emulsion, having oily particles dispersed in a continuous aqueous phase, for immunizing an animal, which process comprises mixing
an immunogenic amount of an antigen; and
an adjuvant prepared according to any one of the Claims 1-19.

## Patentansprüche (Patentansprüche für folgende(n) Vertragsstaat(en): AT, BE, CH, DE, FR, GB, IT, LI, LU, NL, SE)

1. Adjuvans in Form einer Emulsion, die ölige, in einer kontinuierlichen wäßrigen Phase dispergierte Partikel aufweist, zum Potenzieren der Immunogenität eines Antigens, wobei das Adjuvans umfaßt:
eine emulsionsbildende Menge eines nichttoxischen N,N,N',N'-Tetra(polyoxypropylen-polyoxyethylen)-1,2-diaminoethan-Blockcopolymers ("Tetrapolyol") oder eines Polyoxvpropylen-polyoxyethylen ("POP-POE")-Blockcopolymers; und eine immunpotenzierende Menge eines Glykopeptids, worin im wesentlichen alle öligen Partikel einen Durchmesser von weniger als etwa 800 nm aufweisen.

2. Adjuvans nach Anspruch 1 in Form einer Emulsion, die ölige, in einer kontinuierlichen wäßrigen Phase dispergierte Partikel aufweist, zum Potenzieren der Immunogenität eines Antigens, wobei das Adjuvans umfaßt:
eine emulsionsbildende Menge eines nichttoxischen Tetrapolyols;
gegebenenfalls eine emulsionsbildende Menge eines nichttoxischen metabolisierbaren Öls;
eine emulsionsstabilisierende Menge eines Tensids auf Glykolether-Basis;
Wasser oder wäßrige Lösung; und
eine immunpotenzierende Menge eines Muramyldipeptid Derivats der Formel I 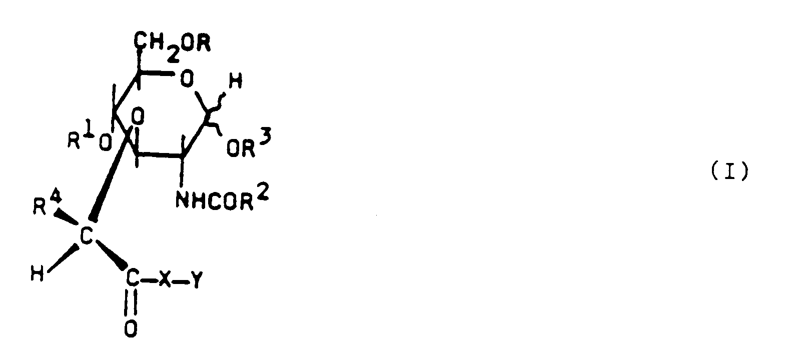 und die pharmazeutisch annehmbaren Salze davon, worin R und R₁ jeweils unabhängig für H oder Acyl mit 1 bis 22 Kohlenstoffatomen stehen;
R₂ für Alkyl oder Aryl, gegebenenfalls substituiert durch Halogen, Nitro oder Niederalkyl, steht;
R₃ für H, Alkyl oder Aryl steht;
R₄ für H oder Niederalkyl steht;
X L-Alanyl, L-α-Aminobutyryl, L-Arginyl, L-Asparginyl, L-Aspartyl, L-Cysteinyl, L-Glutaminyl, L-Glutamyl, Glycyl, L-Histidyl, L-Hydroxyprolyl, L-Isoleucyl, L-Leucyl, L-Lysyl, L-Methionyl, L-Ornithinyl, L-Phenylalanyl, L-Prolyl, L-Seryl, L-Threonyl, L-Tyrosyl, L-Tryptophanyl oder L-Valyl ist und Y D-Glutamin, D-Isoglutamin oder D-Isoasparagin ist.

3. Adjuvans nach Anspruch 1 oder 2, worin das Tetrapolyol Tetronic® 1501 ist, welches ein durchschnittliches Molekulargewicht des POP-Teils von 6500-7000 und einen POE-Prozentanteil von 10% aufweist.

4. Adjuvans nach Anspruch 3, welches ein nichttoxisches metabolisierbares Öl einschließt, worin das Öl Squalen oder Squalan ist.

5. Adjuvans nach Anspruch 3 oder 4, worin das Tensid auf Glykolether-Basis Polysorbat 80 ist.

6. Adjuvans nach Anspruch 3, 4 oder 5, worin das Wasser oder die wäßrige Lösung isotonische gepufferte Kochsalzlösung umfaßt.

7. Adjuvans nach irgendeinem der Ansprüche 1-6, worin im wesentlichen alle öligen Partikel einen Durchmesser von weniger als etwa 300 nm aufweisen.

8. Adjuvans nach irgendeinem der Ansprüche 2-7, worin das Muramyldipeptid Derivat der Formel I:
N-Acetylmuramyl-L-threonyl-D-isoglutamin,
N-Acetylmuramyl-L-α-aminobutyryl-D-isoglutamin,
6-0-Stearoyl-N-acetylmuramyl-L-α-aminobutyryl-D-isoglutamin,
N-Acetylmuramyl-L-valyl-D-isoglutamin,
N-Acetylmuramyl-L-alanyl-D-isoglutamin
N-Acetyl-desmethylmuramyl-L-alanyl-D-isoglutamin,
N-Acetylmuramyl-L-alanyl-D-glutaminbutylester,
N-Acetylmuramyl-L-seryl-D-isoglutamin oder
N-Butyrylmuramyl-L-α-aminobutyryl-D-isoglutamin ist.

9. Adjuvans nach irgendeinem der vorhergehenden Ansprüche in Form einer Emulsion vom Öl-in-Wasser-Typ, die ölige, in einer kontinuierlichen wäßrigen Phase dispergierte Partikel aufweist, zum Potenzieren der Immunogenität eines Antigens, wobei das Adjuvans umfaßt:
ein nichttoxisches Tetrapolyol in einer Menge von 0,2 bis 49%;
ein nichttoxisches metabolisierbares Öl in einer Menge von 0-15%;
ein Tensid auf Glykolether-Basis in einer Menge von 0,05-5%;
Wasser oder wäßrige Lösung; und
0,0001-10% eines Muramyldipeptid-Derivats der Formel I.

10. Adjuvans nach Anspruch 9 in Form einer Emulsion vom Öl-in-Wasser-Typ, die ölige, in einer kontinuierlichen wäßrigen Phase dispergierte Partikel aufweist, zum Potenzieren der Immunogenität eines Antigens, wobei das Adjuvans umfaßt:
Tetronic® 1501 in einer Menge von 1-10%;
Squalan oder Squalen in einer Menge von 1-10%;
Polysorbat 80 in einer Menge von etwa 0,2%;
isotonische gepufferte Kochsalzlösung; und
0,0001-10% N-Acetylmuramyl-L-threonyl-D-isoglutamin.

11. Adjuvans nach Anspruch 1 in Form einer Emulsion vom Öl-in-Wasser-Typ, die ölige, in einer kontinuierlichen wäßrigen Phase dispergierte Partikel aufweist, zum Potenzieren der Immunogenität eines Antigens, wobei das Adjuvans umfaßt:
eine emulsionsbildende Menge eines nichttoxischen POP-POE-Blockcopolymers;
gegebenenfalls eine emulsionsbildende Menge eines nichttoxischen metabolisierbaren Öls;
eine emulsionsstabilisierende Menge eines Tensids auf Glykolether-Basis;
Wasser oder wäßrige Lösung; und
eine immunpotenzierende Menge eines Muramyldipeptid-Derivats der Formel I 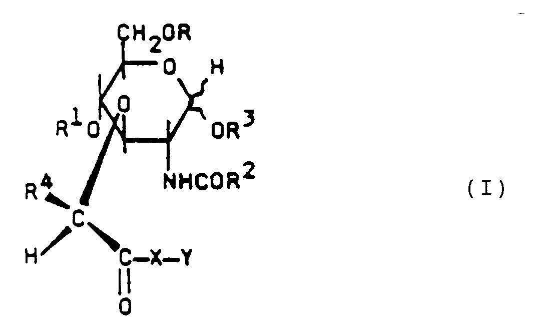 und die pharmazeutisch annehmbaren Salze davon, worin
R und R₁ jeweils unabhängig H oder Acyl mit 1 bis 22 Kohlenstoffatomen darstellen;
R₂ Alkyl oder Aryl, gegebenenfalls substituiert durch Halogen, Nitro oder Niederalkyl darstellt;
R₃ für H, Alkyl oder Aryl steht;
R₄ für H oder Niederalkyl steht;
X L-Alanyl, L-α-Aminobutyryl, L-Arginyl, L-Asparginyl, L-Aspartyl, L-Cysteinyl, L-Glutaminyl, L-Glutamyl, Glycyl, L-Histidyl, L-Hydroxypropyl, L-Isoleucyl, L-Leucyl, L-Lysyl, L-Methionyl, L-Ornithinyl, L-Phenylalanyl, L-Prolyl, L-Seryl, L-Threonyl, L-Tyrosyl, L-Tryptophanyl oder L-Valyl ist;
und
Y D-Glutamin, D-Isoglutamin oder D-Isoasparagin ist, worin im wesentlichen alle öligen Partikel einen Durchmesser von weniger als etwa 800 nm, vorzugsweise weniger als etwa 300 nm, aufweisen.

12. Adjuvans nach Anspruch 11, worin das nichttoxische POP-POE-Blockcopolymer Pluronic® L121 ist, worin das durchschnittliche Molekulargewicht des POP-Teils 4000 und der Prozentanteil an POE 10% beträgt.

13. Adjuvans nach Anspruch 11 oder 12, worin das Muramyldipeptid-Derivat der Formel I N-Acetylmuramyl-L-threonyl-D-isoglutamin ist.

14. Adjuvans nach irgendeinem der Ansprüche 11-13, welches ein nichttoxisches metabolisierbares Öl einschließt, worin das Öl Squalen oder Squalan ist.

15. Adjuvans nach irgendeinem der Ansprüche 11-14, worin das Tensid auf Glykolether-Basis Polysorbat 80 ist.

16. Adjuvans nach irgendeinem der Ansprüche 11-15, worin das Wasser oder die wäßrige Lösung isotonische gepufferte Kochsalzlösung umfaßt.

17. Adjuvans nach Anspruch 11 in Form einer Emulsion vom Öl-in-Wasser-Typ, die ölige, in einer kontinuierlichen wäßrigen Phase dispergierte Partikel aufweist, zum Potenzieren der Immunogenität eines Antigens, wobei das Adjuvans umfaßt:
ein nichttoxisches POP-POE-Blockcopolymer in einer Menge von 0,2 bis 49%;
ein nichttoxisches metabolisierbares Öl in einer Menge von 0-15%;
ein Tensid auf Glykolether-Basis in einer Menge von 0,05-5%;
Wasser oder wäßrige Lösung; und
0,0001-10% eines Muramyldipeptid-Derivats der Formel I, worin im wesentlichen alle öligen Partikel einen Durchmesser von weniger als etwa 800 nm aufweisen.

18. Adjuvans nach Anspruch 17 in Form einer Emulsion vom Öl-in-Wasser-Typ, die ölige, in einer kontinuierlichen wäßrigen Phase dispergierte Partikel aufweist, zum Potenzieren der Immunogenität eines Antigens, wobei das Adjuvans umfaßt:
Pluronic® L121 in einer Menge von 1-10%;
Squalan oder Squalen in einer Menge von 1-10%;
Polysorbat 80 in einer Menge von etwa 0,2%;
isotonische gepufferte Kochsalzlösung; und
0,0001-10% N-Acetylmuramyl-L-threonyl-D-isoglutamin, worin im wesentlichen alle öligen Partikel einen Durchmesser von weniger als etwa 800 nm aufweisen.

19. Vaccine in Form einer Emulsion vom Öl-in-Wasser-Typ, die ölige, in einer kontinuierlichen wäßrigen Phase dispergierte Partikel aufweist, zum Immunisieren eines Tiers, wobei die Vaccine umfaßt:
eine immunogene Menge eines Antigens; und
ein Adjuvans nach irgendeinem der Ansprüche 1-18.

20. Verfahren zum Herstellen des Adjuvans nach irgendeinem der Ansprüche 1-18, wobei das Verfahren das Zusammenmischen der wäßrigen Phase und der emulsionsbildenden Menge des nichttoxischen Tetrapolyols oder des POP-POE-Blockcopolymers umfaßt, um eine Emulsion zu bilden, worin im wesentlichen alle öligen Partikel einen Durchmesser von weniger als etwa 800 nm aufweisen.

21. Verfahren nach Anspruch 20, worin das Glykopeptid der Mischung zugegeben wird, nachdem die Emulsion gebildet worden ist.

22. Verfahren zum Herstellen der Vaccine nach Anspruch 19, wobei das Verfahren das Mischen des Antigens mit dem Adjuvans umfaßt.

23. Reagenzsatz zur extemporierten Herstellung eines Adjuvans nach irgendeinem der Ansprüche 1-18, wobei der Reagenzsatz umfaßt:
einen ersten Behälter, der die Emulsion des Tetrapolyols oder POP-POE Polymers in der wäßrigen Phase enthält, bei der im wesentlichen alle öligen Partikel einen Durchmesser von weniger als etwa 800 nm aufweisen; und
einen zweiten Behälter, der das Glykopeptid enthält.

24. Reagenzsatz nach Anspruch 23 zur extemporierten Herstellung eines Adjuvans der Erfindung, wobei der Reagenzsatz umfaßt:
einen ersten Behälter, der eine Emulsion vom Öl-in-Wasser-Typ enthält, welche ölige, in einer kontinuierlichen wäßrigen Phase dispergierte Partikel aufweist, wobei die Emulsion Tetronic® 1501 oder Pluronic® L121, Squalan oder Squalen, Polysorbat 80 und isotonische gepufferte Kochsalzlösung umfaßt, wobei alle öligen Partikel einen Durchmesser von weniger als etwa 800 nm aufweisen; und
einen zweiten Behälter, der N-Acetylmuramyl-L-threonyl-D-isoglutamin enthält;
wobei die Konzentrationen der Komponenten in jedem Behälter so ausgewählt sind, daß die Kombination der Inhalte beider Behälter eine Tetronic® 1501 oder Pluronic® L121 in einer Menge von 1-30%, Squalan oder Squalen in einer Menge von 1-30%, Polysorbat 80 in einer Menge von etwa 0,2-5%, 0,0001-30% N-Acetylmuramyl-L-threonyl-D-isoglutamin und isotonische gepufferte Kochsalzlösung umfassende Formulierung ergibt.

25. Reagenzsatz zur extemporierten Herstellung einer Vaccine nach Anspruch 19, wobei der Reagenzsatz umfaßt:
einen ersten Behälter, der die Emulsion des Tetrapolyols oder POP-POE-Blockcopolymers in der wäßrigen Phase enthält, worin im wesentlichen alle öligen Partikel einen Durchmesser von weniger als etwa 800 nm aufweisen; und
einen zweiten Behälter, der das Antigen enthält; wobei das Glykopeptid in einem dritten Behälter oder in dem ersten oder zweiten Behälter anwesend sein kann.

26. Reagenzsatz nach Anspruch 25 zur extemporierten Herstellung einer Vaccine der Erfindung, wobei der Reagenzsatz umfaßt:
einen ersten Behälter, der eine Emulsion vom Öl-in-Wasser-Typ enthält, welche ölige, in einer kontinuierlichen wäßrigen Phase dispergierte Partikel aufweist, wobei die Emulsion Tetronic® 1501 oder Pluronic® L121, Squalan oder Squalen, Polysorbat 80 und isotonische gepufferte Kochsalzlösung umfaßt, wobei alle öligen Partikel einen Durchmesser von weniger als etwa 800 nm aufweisen; und
einen zweiten Behälter, der N-Acetylmuramyl-L-threonyl-D-isoglutamin und eine immunogene Menge eines Antigens enthält,
wobei die Konzentrationen der Komponenten in jedem Behälter so ausgewählt sind, daß die Kombination der Inhalte beider Behälter eine Tetronic® 1501 oder Pluronic® L121 in einer Menge von 1-10%, Squalan oder Squalen in einer Menge von 1-10%, Polysorbat 80 in einer Menge von etwa 0,2%, 0,0001-10% N-Acetylmuramyl-L-threonyl-D-isoglutamin, eine immunogene Menge eines Antigens und isotonische gepufferte Kochsalzlösung umfassende Formulierung ergibt.

## Patentansprüche (Patentansprüche für folgende(n) Vertragsstaat(en): ES, GR)

1. Verfahren zur Herstellung eines Adjuvans in Form einer Emulsion, die ölige, in einer kontinuierlichen wäßrigen Phase dispergierte Partikel aufweist, zum Potenzieren der Immunogenität eines Antigens, wobei das Adjuvans umfaßt:
eine emulsionsbildende Menge eines nichttoxischen N,N,N',N'-Tetra(polyoxypropylen-polyoxyethylen)-1,2-diaminoethan-Blockcopolymers ("Tetrapolyol") oder eines Polyoxypropylen-polyoxyethylen ("POP-POE")-Blockcopolymers; und eine immunpotenzierende Menge eines Glykopeptids; worin im wesentlichen alle öligen Partikel einen Durchmesser von weniger als etwa 800 nm aufweisen, welches Verfahren das Zusammenmischen der wäßrigen Phase und des Tetrapolyol- oder POP-POE-Blockcopolymers umfaßt.

2. Verfahren nach Anspruch 1 zur Herstellung eines Adjuvans in Form einer Emulsion, die ölige, in einer kontinuierlichen wäßrigen Phase dispergierte Partikel aufweist, zum Potenzieren der Immunogenität eines Antigens, wobei das Adjuvans umfaßt:
eine emulsionsbildende Menge eines nichttoxischen Tetrapolyols;
gegebenenfalls eine emulsionsbildende Menge eines nichttoxischen metabolisierbaren Öls;
eine emulsionsstabilisierende Menge eines Tensids auf Glykolether-Basis;
Wasser oder wäßrige Lösung; und
eine immunpotenzierende Menge eines Muramyldipeptid-Derivats der Formel I 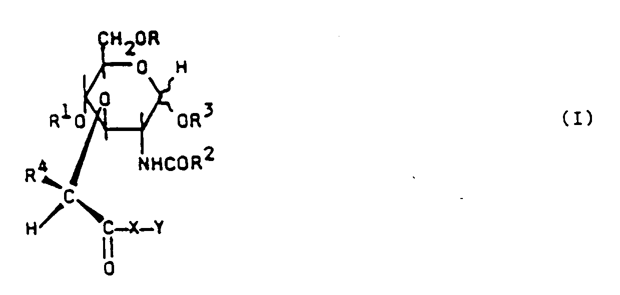 und die pharmazeutisch annehmbaren Salze davon, worin
R und R₁ jeweils unabhängig für H oder Acyl mit 1 bis 22 Kohlenstoffatomen stehen;
R₂ für Alkyl oder Aryl, gegebenenfalls substituiert durch Halogen, Nitro oder Niederalkyl, steht;
R₃ für H, Alkyl oder Aryl steht;
R₄ für H oder Niederalkyl steht;
X L-Alanyl, L-α-Aminobutyryl, L-Arginyl, L-Asparginyl, L-Aspartyl, L-Cysteinyl, L-Glutaminyl, L-Glutamyl, Glycyl, L-Histidyl, L-Hydroxyprolyl, L-Isoleucyl, L-Leucyl, L-Lysyl, L-Methionyl, L-Ornithinyl, L-Phenylalanyl, L-Prolyl, L-Seryl, L-Threonyl, L-Tyrosyl, L-Tryptophanyl oder L-Valyl ist und Y D-Glutamin, D-Isoglutamin oder D-Isoasparagin ist.

3. Verfahren nach Anspruch 1 oder 2, worin das Tetrapolyol Tetronic® 1501 ist, welches ein durchschnittliches Molekulargewicht des POP-Teils von 6500-7000 und einen POE-Prozentanteil von 10% aufweist.

4. Verfahren nach Anspruch 3, welches das Zumischen eines nichttoxischen metabolisierbaren Öls einschließt, worin das Öl Squalen oder Squalan ist.

5. Verfahren nach Anspruch 3 oder 4, worin das Tensid auf Glykolether-Basis Polysorbat 80 ist.

6. Verfahren nach Anspruch 3, 4 oder 5, worin das Wasser oder die wäßrige Lösung isotonische gepufferte Kochsalzlösung umfaßt.

7. Verfahren nach irgendeinem der Ansprüche 1-6, worin im wesentlichen alle öligen Partikel einen Durchmesser von weniger als etwa 300 nm aufweisen.

8. Verfahren nach irgendeinem der Ansprüche 2-7, worin das Muramyldipeptid-Derivat der Formel I:
N-Acetylmuramyl-L-threonyl-D-isoglutamin,
N-Acetylmuramyl-L-α-aminobutyryl-D-isoglutamin,
6-0-Stearoyl-N-acetylmuramyl-L-α-aminobutyryl-D-isoglutamin,
N-Acetylmuramyl-L-valyl-D-isoglutamin,
N-Acetylmuramyl-L-alanyl-D-isoglutamin
N-Acetyl-desmethylmuramyl-L-alanyl-D-isoglutamin,
N-Acetylmuramyl-L-alanyl-D-glutaminbutylester,
N-Acetylmuramyl-L-seryl-D-isoglutamin oder
N-Butyrylmuramyl-L-α-aminobutyryl-D-isoglutamin ist.

9. Verfahren nach irgendeinem der vorhergehenden Ansprüche zur Herstellung eines Adjuvans in Form einer Emulsion vom Öl-in-Wasser-Typ, die ölige, in einer kontinuierlichen wäßrigen Phase dispergierte Partikel aufweist, zum Potenzieren der Immunogenität eines Antigens, wobei das Adjuvans umfaßt:
ein nichttoxisches Tetrapolyol in einer Menge von 0,2 bis 49%;
ein nichttoxisches metabolisierbares Öl in einer Menge von 0-15%;
ein Tensid auf Glykolether-Basis in einer Menge von 0,05-5%;
Wasser oder wäßrige Lösung; und
0,0001-10% eines Muramyldipeptid-Derivats der Formel I.

10. Verfahren nach Anspruch 9 zur Herstellung eines Adjuvans in Form einer Emulsion vom Öl-in-Wasser-Typ, die ölige, in einer kontinuierlichen wäßrigen Phase dispergierte Partikel aufweist, zum Potenzieren der Immunogenität eines Antigens, wobei das Adjuvans umfaßt:
Tetronic® 1501 in einer Menge von 1-10%;
Squalan oder Squalen in einer Menge von 1-10%;
Polysorbat 80 in einer Menge von etwa 0,2%;
isotonische gepufferte Kochsalzlösung; und
0,0001-10% N-Acetylmuramyl-L-threonyl-D-isoglutamin.

11. Verfahren nach Anspruch 1 zur Herstellung eines Adjuvans in Form einer Emulsion vom Öl-in-Wasser-Typ, die ölige, in einer kontinuierlichen wäßrigen Phase dispergierte Partikel aufweist, zum Potenzieren der Immunogenität eines Antigens, wobei das Adjuvans umfaßt:
eine emulsionsbildende Menge eines nichttoxischen POP-POE-Blockcopolymers;
gegebenenfalls eine emulsionsbildende Menge eines nichttoxischen metabolisierbaren Öls;
eine emulsionsstabilisierende Menge eines Tensids auf Glykolether-Basis;
Wasser oder wäßrige Lösung; und
eine immunpotenzierende Menge eines Muramyldipeptid-Derivats der Formel I 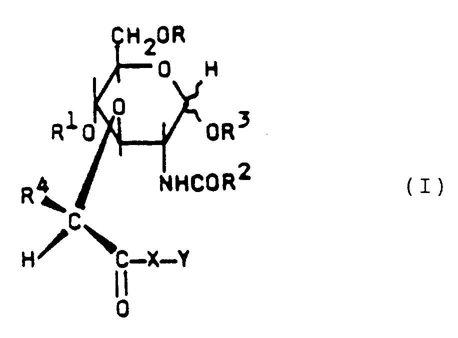 und die pharmazeutisch annehmbaren Salze davon, worin
R und R₁ jeweils unabhängig H oder Acyl mit 1 bis 22 Kohlenstoffatomen darstellen;
R₂ Alkyl oder Aryl, gegebenenfalls substituiert durch Halogen, Nitro oder Niederalkyl darstellt;
R₃ für H, Alkyl oder Aryl steht;
R₄ für H oder Niederalkyl steht;
X L-Alanyl, L-α-Aminobutyry1, L-Arginyl, L-Asparginyl, L-Aspartyl, L-Cysteinyl, L-Glutaminyl, L-Glutamyl, Glycyl, L-Histidyl, L-Hydroxypropyl, L-Isoleucyl, L-Leucyl, L-Lysyl, L-Methionyl, L-Ornithinyl, L-Phenylalanyl, L-Prolyl, L-Seryl, L-Threonyl, L-Tyrosyl, L-Tryptophanyl oder L-Valyl ist; und
Y D-Glutamin, D-Isoglutamin oder D-Isoasparagin ist, worin im wesentlichen alle öligen Partikel einen Durchmesser von weniger als etwa 800 nm, vorzugsweise weniger als etwa 300 nm, aufweisen.

12. Verfahren nach Anspruch 11, worin das nichttoxische POP-POE-Blockcopolymer Pluronic® L121 ist, worin das durchschnittliche Molekulargewicht des POP-Teils 4000 und der Prozentanteil an POE 10% beträgt.

13. Verfahren nach Anspruch 11 oder 12, worin das Muramyldipeptid-Derivat der Formel I N-Acetylmuramyl-L-threonyl-D-isoglutamin ist.

14. Verfahren nach irgendeinem der Ansprüche 11-13, welches einschließt das Zumischen eines nichttoxischen metabolisierbare Öls, worin das Öl Squalen oder Squalan ist.

15. Verfahren nach irgendeinem der Ansprüche 11-14, worin das Tensid auf Glykolether-Basis Polysorbat 80 ist.

16. Verfahren nach irgendeinem der Ansprüche 11-15, worin das Wasser oder die wäßrige Lösung isotonische gepufferte Kochsalzlösung umfaßt.

17. Verfahren nach Anspruch 11 zur Herstellung eines Adjuvans in Form einer Emulsion vom Öl-in-Wasser-Typ, die ölige, in einer kontinuierlichen wäßrigen Phase dispergierte Partikel aufweist, zum Potenzieren der Immunogenität eines Antigens, wobei das Adjuvans umfaßt:
ein nichttoxisches POP-POE-Blockcopolymer in einer Menge von 0,2 bis 49%;
ein nichttoxisches metabolisierbares Öl in einer Menge von 0-15%;
ein Tensid auf Glykolether-Basis in einer Menge von 0,05-5%;
Wasser oder wäßrige Lösung; und
0,0001-10% eines Muramyldipeptid-Derivats der Formel I, worin im wesentlichen alle öligen Partikel einen Durchmesser von weniger als etwa 800 nm aufweisen.

18. Verfahren nach Anspruch 17 zur Herstellung eines Adjuvans in Form einer Emulsion vom Öl-in-Wasser-Typ, die ölige, in einer kontinuierlichen wäßrigen Phase dispergierte Partikel aufweist, zum Potenzieren der Immunogenität eines Antigens, wobei das Adjuvans umfaßt:
Pluronic® L121 in einer Menge von 1-10%;
Squalan oder Squalen in einer Menge von 1-10%;
Polysorbat 80 in einer Menge von etwa 0,2%;
isotonische gepufferte Kochsalzlösung; und
0,0001-10% N-Acetylmuramyl-L-threonyl-D-isoglutamin, worin im wesentlichen alle öligen Partikel einen Durchmesser von weniger als etwa 800 nm aufweisen.

19. Verfahren nach irgendeinem der Ansprüche 1-18, worin das Glykopeptid der Mischung zugegeben wird, nachdem die Emulsion gebildet worden ist.

20. Verfahren zur Herstellung einer Vaccine in Form einer Emulsion vom Öl-in-Wasser-Typ, die ölige, in einer kontinuierlichen wäßrigen Phase dispergierte Partikel aufweist, zum Immunisieren eines Tiers, welches Verfahren umfaßt das Mischen
einer immunogene Mengen eines Antigens; und
eines nach irgendeinem der Ansprüche 1-19 hergestellten Adjuvans.

## Revendications (Revendications pour l'(les) Etat(s) contractant(s) suivant(s): AT, BE, CH, DE, FR, GB, IT, LI, LU, NL, SE)

1. Adjuvant sous forme d'une émulsion possédant des particules huileuses dispersées dans une phase aqueuse continue, pour la potentialisation de l'immunogénicité d'un antigène, adjuvant qui comprend :
une quantité, à effet de formation d'une émulsion, d'un polymère séquencé N,N,N',N'-tétra(polyoxypropylène-polyoxyéthylène)-1,2-diaminoéthane non toxique ("tétrapolyol") ou d'un polymère séquencé polyoxypropylène-polyoxyéthylène ("POP-POE") ; et
une quantité immunopotentialisatrice d'un glycopeptide ;
dans lequel pratiquement la totalité desdites particules huileuses possèdent un diamètre inférieur à environ 800 nm.

2. Adjuvant suivant la revendication 1, sous forme d'une émulsion possédant des particules huileuses dispersées dans une phase aqueuse continue, pour la potentialisation de l'immunogénicité d'un antigène, adjuvant qui comprend :
une quantité, à effet de formation d'une émulsion, d'un tétrapolyol non toxique ;
facultativement, une quantité, à effet de formation d'une émulsion, d'une huile métabolisable non toxique ;
une quantité, à effet de stabilisation de l'émulsion, d'un surfactant à base d'un éther de glycol ;
de l'eau ou une solution aqueuse ; et
une quantité immunopotentialisatrice d'un dérivé de muramyldipeptide de formule I 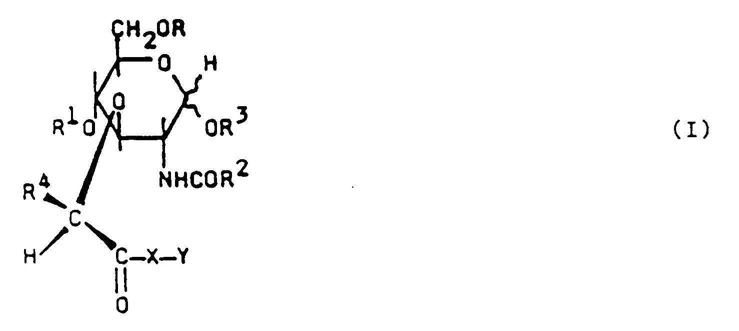 ou d'un de ses sels pharmaceutiquement acceptables,
formule dans laquelle
R et R₁ représentent chacun indépendamment H ou un groupe acyle ayant 1 à 22 atomes de carbone ;
R₂ représente un groupe alkyle ou aryle, facultativement substitué avec un groupe halogéno, nitro ou alkyle inférieur ;
R₃ représente H, un groupe alkyle ou aryle ;
R₄ représente H ou un groupe alkyle inférieur ;
X représente un groupe L-alanyle, L-α-aminobutyryle, L-arginyle, L-asparginyle, L-aspartyle, L-cystéinyle, L-glutaminyle, L-glutamyle, glycyle, L-histidyle, L-hydroxyprolyle, L-isoleucyle, L-leucyle, L-lysyle, L-méthionyle, L-ornithinyle, L-phénylalanyle, L-propyle, L-séryle, L-thréonyle, L-tyrosyle, L-tryptophanyle ou L-valyle ; et
Y représente un groupe D-glutamine, D-isoglutamine ou D-isoasparagine.

3. Adjuvant suivant la revendication 1 ou 2, dans lequel le tétra-polyol est le Tetronic® 1501 qui possède un poids moléculaire moyen de la section POP égal A 6500-7000 et un pourcentage de POE égal à 10 %.

4. Adjuvant suivant la revendication 3, qui comprend une huile métabolisable non toxique, dans lequel ladite huile est le squalène ou le squalane.

5. Adjuvant suivant la revendication 3 ou 4, dans lequel le surfactant à base d'un éther de glycol est le polysorbate 80.

6. Adjuvant suivant la revendication 3, 4 ou 5, dans lequel la solution aqueuse consiste en sérum physiologique tamponné isotonique.

7. Adjuvant suivant l'une quelconque des revendications 1 à 6, dans lequel pratiquement la totalité des particules huileuses possèdent un diamètre inférieur à environ 300 nm.

8. Adjuvant suivant l'une quelconque des revendications 2 à 7, dans lequel le dérivé de muramyldipeptide de formule I est :
la N-acétylmuramyl-L-thréonyl-D-isoglutamine,
la N-acétylmuramyl-L-α-aminobutyryl-D-isoglutamine,
la 6-0-stéaroyl-N-acétylmuramyl-L-α-aminobutyryl-D-isoglutamine,
la N-acétylmuramyl-L-valyl-D-isoglutamine,
la N-acétylmuramyl-L-alanyl-D-isoglutamine,
la N-acétyl-desméthylmuramyl-L-alanyl-D-isoglutamine,
l'ester butylique de N-acétylmuramyl-L-alanyl-D-glutamine,
la N-acétylmuramyl-L-séryl-D-isoglutamine ou
la N-butyrylmuramyl-L-α-aminobutyryl-D-isoglutamine.

9. Adjuvant suivant l'une quelconque des revendications précédentes, sous forme d'une émulsion du type huile-dans-eau, possédant des particules huileuses dispersées dans une phase aqueuse continue, pour la potentialisation de l'immunogénicité d'un antigène, adjuvant qui comprend :
un tétrapolyol non toxique en une quantité de 0,2 à 49 % ;
une huile métabolisable non toxique en une quantité de 0 à 15 % ;
un surfactant à base d'un éther de glycol, en une quantité de 0,05 à 5 % ;
de l'eau ou une solution aqueuse ; et
0,0001 à 10 % d'un dérivé de muramyldipeptide de formule I.

10. Adjuvant suivant la revendication 9, sous forme d'une émulsion du type huile-dans-eau, possédant des particules huileuses dispersées dans une phase aqueuse continue, pour la potentialisation de l'immunogénicité d'un antigène, adjuvant qui comprend :
du Tetronic® 1501 en une quantité de 1 à 10 % ;
du squalane ou squalène en une quantité de 1 à 10 % ;
du polysorbate 80 en une quantité d'environ 0,2 % ;
du sérum physiologique tamponné isotonique ; et
0,0001 à 10 % de N-acétylmuramyl-L-thréonyl-D-isoglutamine.

11. Adjuvant suivant la revendication 1, sous forme d'une émulsion du type huile-dans-eau, possédant des particules huileuses dispersées dans une phase aqueuse continue, pour la potentialisation de l'immunogénicité d'un antigène, adjuvant qui comprend :
une quantité, à effet de formation d'une émulsion, d'un polymère séquencé POP-POE non toxique ;
facultativement, une quantité, à effet de formation d'une émulsion, d'une huile métabolisable non toxique ;
une quantité, à effet de stabilisation de l'émulsion, d'un surfactant à base d'un éther de glycol ;
de l'eau ou une solution aqueuse ; et
une quantité immunopotentialisatrice d'un dérivé de muramyldipeptide de formule I 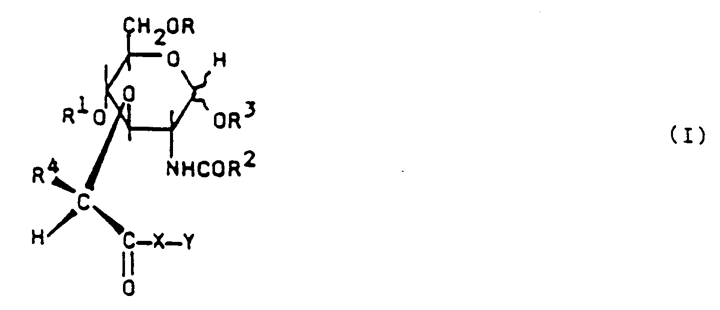 ou d'un des ses sels pharmaceutiquement acceptables,
formule dans laquelle
R et R₁ représentent chacun indépendamment H ou un groupe acyle ayant 1 à 22 atomes de carbone ;
R₂ représente un groupe alkyle ou aryle, facultativement substitué avec un groupe halogéno, nitro ou alkyle inférieur ;
R₃ représente H, un groupe alkyle ou aryle ;
R₄ représente H ou un groupe alkyle inférieur ;
X représente un groupe L-alanyle, L-α-aminobutyryle, L-arginyle, L-asparginyle, L-aspartyle, L-cystéinyle, L-glutaminyle, L-glutamyle, glycyle, L-histidyle, L-hydroxyprolyle, L-isoleucyle, L-leucyle, L-lysyle, L-méthionyle, L-ornithinyle, L-phénylalanyle, L-propyle, L-séryle, L-thréonyle, L-tyrosyle, L-tryptophanyle ou L-valyle ; et
Y représente un groupe D-glutamine, D-isoglutamine ou D-isoasparagine,
dans lequel pratiquement la totalité desdites particules huileuses possèdent un diamètre inférieur à environ 800 nm, de préférence inférieur à environ 300 nm.

12. Adjuvant suivant la revendication 11, dans lequel le polymère séquencé POP-POE non toxique est le Pluronic® L121 dans lequel le poids moléculaire moyen de la section POB est égal à 4000 et le pourcentage de POE est égal à 10 %.

13. Adjuvant suivant la revendication 11 ou 12, dans lequel le dérivé de muramyl-dipeptide de formule I est la N-acétylmuramyl-L-thréonyl-D-isoglutamine.

14. Adjuvant suivant l'une quelconque des revendications 11 à 13, qui comprend une huile métabolisable non toxique, dans lequel ladite huile est le squalène ou le squalane.

15. Adjuvant suivant l'une quelconque des revendications 11 à 14, dans lequel le surfactant à base d'un éther de glycol est le polysorbate 80.

16. Adjuvant suivant l'une quelconque des revendications 11 à 15, dans lequel la solution aqueuse consiste en sérum physiologique tamponné isotonique.

17. Adjuvant suivant la revendication 11, sous forme d'une émulsion du type huile-dans-eau, possédant des particules huileuses dispersées dans une phase continue, pour la potentialisation de l'immunogénicité d'un antigène, adjuvant qui comprend :
un polymère séquencé POP-POE non toxique en une quantité de 0,2 à 49 % ;
une huile métabolisable non toxique en une quantité de 0 à 15 % ;
un surfactant à base d'un éther de glycol en une quantité de 0,05 à 5 % ;
de l'eau ou une solution aqueuse ; et
0,0001 à 10 % d'un dérivé de muramyldipeptide de formule I,
dans lequel pratiquement la totalité desdites particules huileuses possèdent un diamètre inférieur à environ 800 nm.

18. Adjuvant suivant la revendication 17, sous forme d'une émulsion du type huile-dans-eau, possédant des particules huileuses dispersées dans une phase aqueuse continue, pour la potentialisation de l'immunogénicité d'un antigène, adjuvant qui comprend :
du Pluronic® L121 en une quantité de 1 à 10 % ;
du squalane ou squalène en une quantité de 1 à 10 % ;
du polysorbate 80 en une quantité d'environ 0,2 % ;
du sérum physiologique tamponné isotonique ; et
0,0001 à 10 % de N-acétylmuramyl-L-thréonyl-D-isoglutamine,
dans lequel pratiquement la totalité desdites particules huileuses possèdent un diamètre inférieur à environ 800 nm.

19. Vaccin sous forme d'une émulsion du type huile-dans-eau, possédant des particules huileuses dispersées dans une phase aqueuse continue, pour l'immunisation d'un animal, vaccin qui comprend :
une quantité immunogène d'un antigène ; et
un adjuvant suivant l'une quelconque des revendications 1 à 18.

20. Procédé de préparation de l'adjuvant suivant l'une quelconque des revendications 1 à 18, procédé qui comprend l'étape consistant à mélanger ensemble la phase aqueuse et la quantité, à effet de formation d'une émulsion, du tétrapolyol ou du polymère séquencé POP-POE non toxique de manière à former une émulsion, dans lequel pratiquement toutes les particules huileuses possèdent un diamètre inférieur à environ 800 nm.

21. Procédé suivant la revendication 20, dans lequel le glycopeptide est ajouté au mélange après formation de l'émulsion.

22. Procédé de préparation du vaccin suivant la revendication 19, procédé qui comprend le mélange de l'antigêne à l'adjuvant.

23. Kit pour la préparation extemporanée d'un adjuvant suivant l'une quelconque des revendications 1 à 18, kit qui comprend :
un premier récipient contenant l'émulsion du tétrapolyol ou du polymère POP-POE dans la phase aqueuse, dans laquelle pratiquement toutes les particules huileuses possèdent un diamètre inférieur à environ 800 nm ; et
un second récipient contenant le glycopeptide.

24. Kit suivant la revendication 23 pour la préparation extemporanée d'un adjuvant conforme à la présente invention, kit qui comprend :
un premier récipient contenant une émulsion du type huile-dans-eeau, possédant des particules huileuses dispersées dans une phase aqueuse continue, dans laquelle ladite émulsion comprend du Tetronic® 1501 ou du Pluronic® L121, du squalane ou du squalène, du polysorbate 80 et du sérum physiologique tamponné isotonique, émulsion dans laquelle pratiquement la totalité desdites particules huileuses possèdent un diamètre inférieur à environ 800 nm ; et
un second récipient contenant de la N-acétylmuramyl-L-thréonyl-D-isoglutamine ;
dans lequel les concentrations des constituants dans chaque récipient sont choisies de telle sorte que l'association des contenus des deux récipients produise une formulation comprenant du Tetronic® 1501 ou du Pluronic® L121 en une quantité de 1 à 30 %, du squalane ou squalène en une quantité de 1 à 30 %, du polysorbate 80 en une quantité d'environ 0,2 à 5 %, 0,0001 à 30 % de N-acétylmuramyl-L-thréonyl-D-isoglutamine, et du sérum physiologique tamponné isotonique.

25. Kit pour la préparation extemporanée d'un vaccin suivant la revendication 19, kit qui comprend :
un premier récipient contenant une émulsion du tétrapolyol ou du polymère séquencé POP-POE dans la phase aqueuse, dans laquelle pratiquement toutes les particules huileuses possèdent un diamètre inférieur à 800 nm ; et
un second récipient contenant l'antigène ;
dans lequel le glycopeptide peut être présent dans un troisième récipient, ou bien dans le premier ou second récipient.

26. Kit suivant la revendication 25 pour la préparation extemporanée d'un vaccin conforme à la présente invention, kit qui comprend :
un premier récipient contenant une émulsion du type huile-dans-eau, possédant des particules huileuses dispersées dans une phase aqueuse continue, ladite émulsion comprenant du Tetronic® 1501 ou du Pluronic® L121, du squalane ou du squalène, du polysorbate 80 et du sérum physiologique tamponné isotonique, émulsion dans laquelle pratiquement la totalité desdites particules huileuses possède un diamètre inférieur à environ 800 nm ; et
un second récipient contenant de la N-acétylmuramyl-L-thréonyl-D-isoglutamine et une quantité immunogène d'un antigène ;
dans lequel les concentrations des constituants dans chaque récipient sont choisies de telle sorte que l'association des contenus des deux récipients produise une formulation comprenant du Tetronic® 1501 ou du Pluronic® L121 en une quantité de 1 à 10 %, du squalane ou squalène en une quantité de 1 à 10 %, du polysorbate 80 en une quantité d'environ 0,2 %, 0,0001 à 10 % de N-acétylmuramyl-L-thréonyl-D-isoglutamine, une quantité immunogène d'un antigène, et du sérum physiologique tamponné isotonique.

## Revendications (Revendications pour l'(les) Etat(s) contractant(s) suivant(s): ES, GR)

1. Procédé de préparation d'un adjuvant sous forme d'une émulsion possédant des particules huileuses dispersées dans une phase aqueuse continue, pour la potentialisation de l'immunogénicité d'un antigène, adjuvant qui comprend :
une quantité, à effet de formation d'une émulsion, d'un polymère séquencé N,N,N',N'tétra(polyoxypropylène-polyoxéthylène)-1,2-diaminoéthane non toxique ("tétrapolyol") ou d'un polymère séquencé polyoxypropylène-polyoxyéthylène ("POP-POE") ; et
une quantité immunopotentialisatrice d'un glycopeptide ;
dans lequel pratiquement la totalité desdites particules huileuses possèdent un diamètre inférieur à environ 800 nm, procédé qui comprend l'étape consistant à mélanger ensemble la phase aqueuse et le polymère séquencé tétrapolyol ou POP-POE.

2. Procédé suivant la revendication 1, pour la préparation d'un adjuvant sous forme d'une émulsion possédant des particules huileuses dispersées dans une phase aqueuse continue, pour la potentialisation de l'immunogénicité d'un antigène, adjuvant qui comprend :
une quantité, à effet de formation d'une émulsion, d'un tétrapolyol non toxique ;
facultativement, une quantité, à effet de formation d'une émulsion, d'une huile métabolisable non toxique ;
une quantité, à effet de stabilisation de l'émulsion, d'un surfactant à base d'un éther de glycol ;
de l'eau ou une solution aqueuse ; et
une quantité immunopotentialisatrice d'un dérivé de muramyldipeptide de formule I 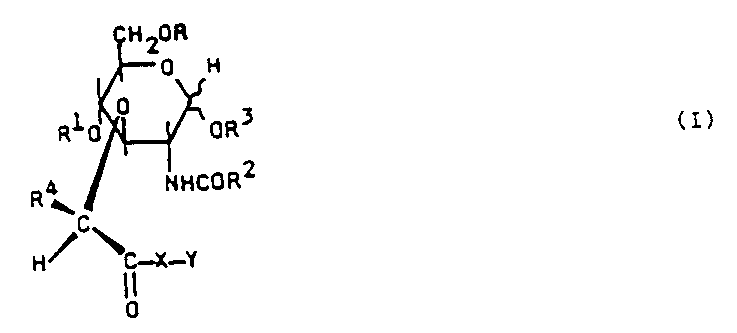 ou d'un de ses sels pharmaceutiquement acceptables,
formule dans laquelle
R et R₁ représentent chacun indépendamment H ou un groupe acyle ayant 1 à 22 atomes de carbone ;
R₂ représente un groupe alkyle ou aryle, facultativement substitué avec un groupe halogéno, nitro ou alkyle inférieur ;
R₃ représente H, un groupe alkyle ou aryle ;
R₄ représente H ou un groupe alkyle inférieur ;
X représente un groupe L-alanyle, L-α-aminobutyryle, L-arginyle, L-asparginyle, L-aspartyle, L-cystéinyle, L-glutaminyle, L-glutamyle, glycyle, L-histidyle, L-hydroxyprolyle, L-isoleucyle, L-leucyle, L-lysyle, L-méthionyle, L-ornithinyle, L-phénylalanyle, L-propyle, L-séryle, L-thréonyle, L-tyrosyle, L-tryptophanyle ou L-valyle ; et
Y représente un groupe D-glutamine, D-isoglutamine ou D-isoasparagine.

3. Procédé suivant la revendication 1 ou 2, dans lequel le tétrapolyol est le Tetronic® 1501 qui possède un poids moléculaire moyen de la section POP égal à 6500-7000 et un pourcentage de POE de 10 %.

4. Procédé suivant la revendication 3, qui comprend le mélange d'une huile métabolisable non toxique, dans lequel ladite huile est le squalène ou le squalane.

5. Procédé suivant la revendication 3 ou 4, dans lequel le surfactant à base d'un éther de glycol est le polysorbate 80.

6. Procédê suivant la revendication 3, 4 ou 5, dans lequel la solution aqueuse consiste en sérum physiologique tamponné isotonique.

7. Procédé suivant l'une quelconque des revendications 1 à 6, dans lequel pratiquement la totalité des particules huileuses possèdent un diamètre inférieur à environ 300 nm.

8. Procédé suivant l'une quelconque des revendications 2 à 7, dans lequel le dérivé de muramyldipeptide de formule I est :
la N-acétylmuramyl-L-thréonyl-D-isoglutamine,
la N-acétylmuramyl-L-α-aminobutyryl-D-isoglutamine,
la 6-0-stéaroyl-N-acétylmuramyl-L-α-aminobutyryl-D-isoglutamine,
la N-acétylmuramyl-L-valyl-D-isoglutamine,
la N-acétylmuramyl-L-alanyl-D-isoglutamine,
la N-acétyl-desméthylmuramyl-L-alanyl-D-isoglutamine,
l'ester butylique de N-acétylmuramyl-L-alanyl-D-glutamine,
la N-acétylmuramyl-L-séryl-D-isoglutamine ou
la N-butyrylmuramyl-L-α-aminobutyryl-D-isoglutamine.

9. Procédé suivant l'une quelconque des revendications précédentes pour la préparation d'un adjuvant sous forme d'une émulsion du type huile-dans-eau, possédant des particules huileuses dispersées dans une phase aqueuse continue, pour la potentialisation de l'immunogénicité d'un antigène, adjuvant qui comprend :
un tétrapolyol non toxique en une quantité de 0,2 à 49 % ;
une huile métabolisable non toxique en une quantité de 0 à 15 % ;
un surfactant à base d'un éther de glycol, en une quantité de 0,05 à 5 % ;
de l'eau ou une solution aqueuse ; et
0,0001 à 10 % d'un dérivé de muramyldipeptide de formule I.

10. Procédé suivant la revendication 9 pour la préparation d'un adjuvant sous forme d'une émulsion du type huile-dans-eau, possédant des particules huileuses dispersées, dans une phase aqueuse continue, pour la potentialisation de l'immunogénicité d'un antigène, adjuvant qui comprend :
du Tetronic® 1501 en une quantité de 1 à 10 % ;
du squalane ou squalène en une quantité de 1 à 10 % ;
du polysorbate 80 en une quantité d'environ 0,2 % ;
du sérum physiologique tamponné isotonique ; et
0,0001 à 10 % de N-acétylmuramyl-L-thréonyl-D-isoglutamine.

11. Procédé suivant la revendication 1, pour la préparation d'un adjuvant sous forme d'une émulsion du type huile-dans-eau, possédant des particules huileuses dispersées dans une phase aqueuse continue, pour la potentialisation de l'immunogénicité d'un antigène, adjuvant qui comprend :
une quantité, A effet de formation d'une émulsion, d'un polymère séquencé POP-POE non toxique ;
facultativement, une quantité, à effet de formation d'une émulsion, d'une huile métabolisable non toxique ;
une quantité, à effet de stabilisation de l'émulsion, d'un surfactant à base d'un éther de glycol ;
de l'eau ou une solution aqueuse ; et
une quantité immunopotentialisatrice d'un dérivé de muramyldipeptide de formule I 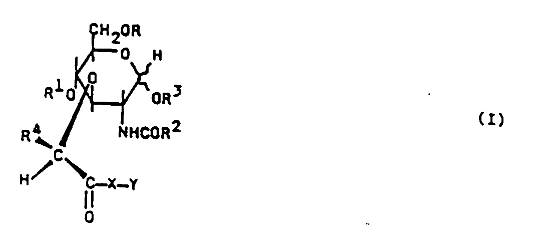 ou d'un des ses sels pharmaceutiquement acceptables,
formule dans laquelle
R et R₁ représentent chacun indépendamment H ou un groupe acyle ayant 1 à 22 atomes de carbone ;
R₂ représente un groupe alkyle ou aryle, facultativement substitué avec un groupe halogéno, nitro ou alkyle inférieur ;
R₃ représente H, un groupe alkyle ou aryle ;
R₄ représente H ou un groupe alkyle inférieur ;
X représente un groupe L-alanyle, L-α-aminobutyryle, L-arginyle, L-asparginyle, L-aspartyle, L-cystéinyle, L-glutaminyle, L-glutamyle, glycyle, L-histidyle, L-hydroxyprolyle, L-isoleucyle, L-leucyle, L-lysyle, L-méthionyle, L-ornithinyle, L-phénylalanyle, L-propyle, L-séryle, L-thréonyle, L-tyrosyle, L-tryptophanyle ou L-valyle ; et
Y représente un groupe D-glutamine, D-isoglutamine ou D-isoasparagine,
dans lequel pratiquement la totalité desdites particules huileuses possèdent un diamètre inférieur à environ 800 nm, de préférence inférieur à environ 300 nm.

12. Procédé suivant la revendication 11, dans lequel le polymère séquencé POP-POE non toxique est le Pluronic® L121 dans lequel le poids moléculaire moyen de la section POP est égal à 4000 et le pourcentage de POE est égal à 10 %.

13. Procédé suivant la revendication 11 ou 12, dans lequel le dérivé de muramyl-dipeptide de formule I est la N-acétylmuramyl-L-thréonyl-D-isoglutamine.

14. Procédé suivant l'une quelconque des revendications 11 à 13, qui comprend le mélange d'une huile métabolisable non toxique, dans lequel ladite huile est le squalène ou le squalane.

15. Procédé suivant l'une quelconque des revendications 11 à 14, dans lequel le surfactant à base d'un éther de glycol est le polysorbate 80.

16. Procédé suivant l'une quelconque des revendications 11 à 15, dans lequel la solution aqueuse consiste en sérum physiologique tamponné isotonique.

17. Procédé suivant la revendication 11 pour la préparation d'un adjuvant sous forme d'une émulsion du type huile-dans-eau, possédant des particules huileuses dispersées dans une phase aqueuse continue, pour la potentialisation de l'immunogénicité d'un antigène, adjuvant qui comprend :
un polymère séquencé POP-POE non toxique en une quantité de 0,2 à 49 % ;
une huile métabolisable non toxique en une quantité de 0 à 15 % ;
un surfactant à base d'un éther de glycol en une quantité de 0,05 à 5 % ;
de l'eau ou une solution aqueuse ; et
0,0001 à 10 % d'un dérivé de muramyldipeptide de formule I,
dans lequel pratiquement la totalité desdites particules huileuses possèdent un diamètre inférieur à environ 800 nm.

18. Procédé suivant la revendication 17 pour la préparation d'un adjuvant sous forme d'une émulsion du type huile-dans-eau, possédant des particules huileuses dispersées dans une phase aqueuse continue, pour la potentialisation de l'immunogénicité d'un antigène, adjuvant qui comprend :
du Pluronic® L121 en une quantité de 1 à 10 % ;
du squalane ou squalène en une quantité de 1 à 10 % ;
du polysorbate 80 en une quantité d'environ 0,2 % ;
du sérum physiologique tamponné isotonique ; et
0,0001 à 10 % de N-acétylmuramyl-L-thréony-D-isoglutamine,
dans lequel pratiquement la totalité desdites particules huileuses possèdent un diamètre inférieur à environ 800 nm.

19. Procédé suivant l'une quelconque des revendications 1 à 18, dans lequel le glycopeptide est ajouté au mélange après formation de l'émulsion.

20. Procédé de préparation d'un vaccin sous forme d'une émulsion du type huile-dans-eau, possédant des particules huileuses dispersées dans une phase aqueuse continue, pour l'immunisation d'un animal, procédé qui comprend le mélange
d'une quantité immunogène d'un antigène ; et
d'un adjuvant préparé suivant l'une quelconque des revendications 1 à 19.
